# EUROPEAN PATENT SPECIFICATION

(11) **EP 3 517 078 B1**
(45) Date of publication and mention of the grant of the patent: **28.10.2020**
(21) Application number: 19153366.0
(22) Date of filing: 23.01.2019
(51) Int. Cl.: A61F 2/46, A61F 2/30

(54) **ORTHOPAEDIC IMPLANT WITH POROUS STRUCTURAL MEMBER**
ORTHOPÄDISCHES IMPLANTAT MIT PORÖSEM STRUKTURELEMENT
IMPLANT ORTHOPÉDIQUE DOTÉ D'UN ÉLÉMENT STRUCTURAL POREUX

(30) Priority: 24.01.2018 US 201815878723
(43) Date of publication of application: 31.07.2019
(73) Proprietor: SMed-TA/TD, LLC, Columbia City, IN 46725 (US)
(72) Inventor: Knapp, Troy D., Hammond, Wisconsin 54015 (US); Nebosky, Paul S., Fort Wayne, Indiana 46807 (US); Stalcup, Gregory C., Columbia City, Indiana 46725 (US)
(74) Representative: Fuchs Patentanwälte Partnerschaft mbB

(56) References cited:
- US-A1- 2011 137 418
- US-A1- 2011 230 970
- US-A1- 2013 190 875

## Description

### BACKGROUND OF THE INVENTION

### 1. Field of the Invention

The present invention relates to orthopaedic devices, and, more particularly, to orthopaedic implants.

### 2. Description of the Related Art

Most orthopaedic implants are formed from a metallic material suitable for a given implant, such as a hip implant, knee implant, glenoid implant, etc. In the case of articulating joints, the implant may include a non-metallic load bearing surface, such as an ultra high molecular weight polyethylene (UHMWPE). The UHMWPE is attached to the metallic body of the implant, and provides the implant with good wear characteristics and low friction.

It is also known to provide an implant with a porous bony ingrowth surface. For example, a hip implant may include a porous surface on the stem which is intended to allow bony ingrowth of the proximal end of the femur bone. Such a porous surface may be in the form of a metal porous surface which is bonded, such as by heat sintering, to the stem of the implant. Examples of porous surfaces of this type include a woven mesh, a fiber mesh and particles. Knee implants are also known that include porous ingrowth surfaces that can bear load from surrounding anatomic structures.

Porous surfaces of the type described above which are used with implants are not typically part of a single structural member with two opposed, external porous surfaces. For example, in a knee implant, the distal surface of the implant can sit on the porous material that is slightly above the substrate material, but the porous material only typically has one external surface for tissue ingrowth. For hip implants, the porous ingrowth surface is usually provided as a coating on a structural component of the implant, such as the stem.

In some orthopaedic applications, such as spinal cages, it is beneficial to have a porous member that extends between two external, load bearing surfaces of the implant. In such arrangements, a cavity is typically formed between the two external surfaces of the implant and filled with a porous ingrowth material, which is typically a natural substance such as cancellous bone tissue. Such an implant is described in U.S. Patent Application No. 2002/0091447 to Shimp et al. One problem with the implant described by Shimp et al. is that harvesting sufficient cancellous bone tissue to fill the cavity is expensive, and host rejection issues can be a concern. Other similar implants that contemplate utilizing natural or synthetic materials are described in U.S. Patent Application Publication No. 2004/0210316 to King et al., and U.S. Patent No. 6,423,095 to Van Hoeck et al. In each of these described implants, the porous material held in the cavity is fairly isolated from bearing load from surrounding anatomic structures after implantation, with external surfaces that are either flush or below the most protruding external surface of the main implant body. This is intentional, as the materials placed in the cavity tend to have significantly lower strength than the implant body. However, isolating the porous ingrowth material from bearing loads from surrounding anatomic structures also decreases the amount of surface area the porous ingrowth material has in contact with the anatomic structures, which can slow down integration of the implant. In addition, the porous materials placed in the cavity are typically resorbable by the body and will not last throughout the life of the implant.

Document US 2011/230970 A1 discloses a spinal implant comprising an anterior wall, a posterior wall and two lateral walls configured to extend between the anterior wall and the posterior wall. The spinal implant further comprises at least one internal chamber generally positioned between the anterior wall, the posterior wall and the two lateral walls, wherein the internal chamber is adapted to receive at least one graft and/or other fill material. In some arrangements, the anterior wall of the spinal implant comprises at least one opening or hole that places the internal chamber in fluid communication with an exterior area or portion of the spinal implant. In one embodiment, at least one of the two lateral walls comprises an access port.

Porous materials in a cavity of an implant may be loaded with one or more types of biological agents, to assist with the healing of nearby anatomical structures or tissues, or to protect against infections, or to fight disease, via the absorption of the agent by the surrounding tissue via the pores of the porous material. However, problems exist with expulsion of the agent from the pores after loading the porous material with the agent and prior to implantation of the implant, and of leakage of the agent through the opening via which the implant was initially loaded, particularly if the agent was loaded into the implant after the implant was positioned in the body.

What is needed in the art is an orthopaedic implant and associated devices and methods that can overcome some of the disadvantages of known implants and methods.

### SUMMARY OF THE INVENTION

In accordance with an aspect of the present invention, there is provided an orthopaedic implant including an implant body having a first surface, a second surface opposite the first surface, and a cavity formed therein that extends through the first surface and the second surface. The implant body is substantially non-porous, and further includes a third surface with at least one first opening formed therethrough to the cavity. The at least one first opening includes an outer portion having an outer diameter and an inner portion having an inner diameter. The outer diameter is greater than the inner diameter. The implant further includes a load bearing member including a substantially porous material held within the cavity, and the substantially porous material is configured to receive, via the at least one first opening, a material agent. The load bearing member has a first contact surface extending out of the cavity past the first surface. The outer portion of the at least one first opening is configured to couple to a tool for receiving, from the tool, the material agent, and the inner portion of the at least one first opening is configured to couple to a plug for preventing the material agent, once received by the substantially porous material via the at least one first opening from the tool, from exiting the substantially porous material via the at least one first opening.

In accordance with an aspect of the present invention, there is provided a tool configured for use with an orthopaedic implant. The orthopaedic implant includes an implant body having a first surface, a second surface opposite the first surface, a cavity formed therein that extends through the first surface and the second surface, the implant body being substantially non-porous and further including a third surface with at least one first opening formed therethrough to the cavity and at least one second opening, the at least one first opening comprising an threaded outer portion having an outer diameter and an threaded inner portion having an inner diameter, the outer diameter greater than the inner diameter. The implant further includes a load bearing member including a substantially porous material held within the cavity, the load bearing member configured to receive, via the at least one first opening, a material agent, the load bearing member having a first contact surface extending out of the cavity past the first surface. The tool includes a tubular assembly having a tubular passage including a first end and a second end, the first end having a means for attachment to the implant body, a plug, and a plunger coupled to the plug. The tubular passage is configured to receive, via the second end, the material agent and the plunger coupled to the plug. The plunger coupled to the plug is configured to slide through the tubular passage for expelling the material agent from the tubular passage into the load bearing member via the at least one first opening. The plunger coupled to the plug is also configured to rotate within the tubular passage for coupling the plug with the first opening to seal the first opening against expulsion of the material agent from the load bearing member via the first opening.

In accordance with an aspect of the present invention, there is provided a method of charging an orthopaedic implant with a material agent. The orthopaedic implant includes an implant body having a first surface, a second surface opposite the first surface, a cavity formed therein that extends through the first surface and the second surface, the implant body being substantially non-porous and further including a third surface with at least one first opening formed therethrough to the cavity and at least one second opening, the at least one first opening comprising an threaded outer portion having an outer diameter and an threaded inner portion having an inner diameter, the outer diameter greater than the inner diameter. The implant further includes a load bearing member including a substantially porous material held within the cavity, the load bearing member configured to receive, via the at least one first opening, a material agent, the load bearing member having a first contact surface extending out of the cavity past the first surface. The method includes coupling a tubular assembly to the third surface of the implant body, wherein the tubular assembly includes a tubular passage having a first end and a second end, and wherein the first end is coupled to the third surface such that said tubular passage is centered on the at least one first opening, placing the material agent into the tubular passage via the second end, sliding a plunger coupled to a plug through the tubular passage via the second end, thereby expelling the material agent from the tubular passage into the load bearing member via the at least one first opening, and rotating the plunger within the tubular passage for coupling the plug with the at least one first opening for sealing the at least one first opening against expulsion of the material agent from the load bearing member via the first opening.

In the method of charging an orthopaedic implant, said tubular assembly can comprise an inserter and a cannula, wherein said inserter includes a tubular portion and an attachment portion having at least one pin, wherein said cannula includes a threaded attachment portion, and wherein coupling said tubular assembly to said third surface of said implant body further comprises:
coupling said at least one pin of said inserter to said at least one second opening of said third surface of said implant body;
sliding said cannula into said tubular portion of said inserter; and
coupling said threaded attachment portion of said cannula to said threaded outer portion of said at least one first opening of said third surface,
wherein said cannula includes said tubular passage.

The method can further comprise:
decoupling said plug from said plunger;
withdrawing said plunger from said tubular passage via said second end;
decoupling said cannula from said threaded outer portion of said at least one first opening of said third surface;
withdrawing said cannula from said tubular portion of said inserter; and
decoupling said inserter from said at least one second opening of said third surface of said implant body.

Further, in the method of charging an orthopaedic implant, if said plunger is coupled to said plug via a breakable joint, said decoupling said plug from said plunger can comprise rotating said plunger within said tubular passage until said breakable joint breaks.

In the method of charging an orthopaedic implant, said material agent can be a flowable material.

Said flowable material can be a bone graft.

Said flowable material can be a bone putty.

Said flowable material can be a therapeutic.

An advantage of the present invention is that the orthopaedic implant can be initially charged with a material agent (the invention as claimed in the attached claims) or after the implant is placed in the body, or re-charged with the material agent during a second surgical (not claimed).

Another advantage of the present invention is that one or more openings of the orthopaedic implant, through which the implant is charged with the material agent, can be sealed against leakage or expulsion of the material agent from the implant.

### BRIEF DESCRIPTION OF THE DRAWINGS

The above-mentioned and other features and advantages of this invention, and the manner of attaining them, will become more apparent and the invention will be better understood by reference to the following description of embodiments of the invention taken in conjunction with the accompanying drawings, wherein:
Fig. 1 is a perspective view of an embodiment of a solid component of a device formed according to the present invention;
Fig. 2 is a perspective view of an embodiment of a porous component of a device formed according to the present invention;
Fig. 3 is a perspective view of a device created from the solid component shown in Fig. 1 and the porous component shown in Fig. 2;
Fig. 4 is a cross-sectional view of a single, continuous layer with porous and solid regions;
Fig. 5 is a perspective view of an embodiment of a spinal cage with windows;
Fig. 6 is a cross-sectional view of the spinal cage shown in Fig. 5 taken along line 6-6;
Fig. 7 is a perspective view of an embodiment of a spinal cage with a ledge or groove;
Fig 8 is a cross-sectional view of the spinal cage shown in Fig. 7 taken along line 8-8;
Fig. 9 is a perspective view of an embodiment of a spinal cage with a two-part solid component that is assembled to contain the porous material;
Fig. 10 is a cross-sectional view of the spinal cage shown in Fig. 9 taken along line 10-10;
Fig. 11 is a perspective view of an embodiment of a spinal cage with laminates perpendicular to an axis of the spinal cage;
Fig. 12 is a perspective view of an embodiment of a spinal cage with laminates parallel to an axis of the spinal cage;
Fig. 13 is a perspective view of an embodiment of a spinal cage with laminates at an angle to an axis of the spinal cage;
Fig. 14 is a perspective view of an embodiment of a spinal cage;
Fig. 15 is a perspective view of another embodiment of a spinal cage;
Fig. 16 is a perspective view of yet another embodiment of a spinal cage;
Fig. 17 is a perspective view of yet another embodiment of a spinal cage;
Fig. 18 is a sectional view of an implant with features for the delivery of therapeutic agents;
Fig. 19 is a sectional view of a tapered implant;
Fig. 20 is a sectional view of another tapered implant;
Fig. 21 is a sectional view of yet another tapered implant;
Fig. 22 is a sectional view of yet another tapered implant;
Fig. 23 is a sectional view of yet another tapered implant;
Fig. 24 is a perspective view of an implant showing teeth that mate with surrounding bone;
Fig. 25 is a side view of the implant shown in Fig. 24;
Fig. 26 is a spinal fusion device;
Fig. 27 is a perspective view of another embodiment of an orthopaedic implant according to the present invention;
Fig. 28 is a side view of the orthopaedic implant shown in Fig. 27;
Fig. 29 is a front view of the orthopaedic implant shown in Figs. 27-28;
Fig. 30 is a perspective view of yet another embodiment of an orthopaedic implant according to the present invention;
Fig. 31 is a side view of the orthopaedic implant shown in Fig. 30;
Fig. 32 is a perspective view of yet another embodiment of an orthopaedic implant according to the present invention;
Fig. 33 is a front view of the orthopaedic implant shown in Fig. 32;
Fig. 34 is a side view of the orthopaedic implant shown in Figs. 32-33;
Fig. 35 is a side view of the orthopaedic implant shown in Figs. 32-34 including an ingrowth material;
Fig. 36 is a perspective view of yet another embodiment of an orthopaedic implant according to the present invention;
Fig. 37 is a side view of the orthopaedic implant shown in Fig. 36;
Fig. 38 is a side view of the orthopaedic implant shown in Figs. 36-37 including an ingrowth material;
Fig. 39 is a perspective view of an orthopaedic implant according to the present invention;
Fig. 40 is a side view of the orthopaedic implant of Fig. 39, according to an embodiment of the present invention;
Fig. 41 is a cross-sectional view of the orthopaedic implant of Fig. 39, with the plug removed, according to an embodiment of the present invention;
Fig. 42 is a top view of the plug of the orthopaedic implant of Fig. 39, according to an embodiment of the present invention;
Fig. 43 is a cross-sectional view of the orthopaedic implant of Fig. 39, including the plug 532, coupled to the first opening for sealing the first opening, according to an embodiment of the present invention;
Fig. 44 illustrates a cross-sectional view of an insertion/delivery (ID) tool coupled to the othopaedic implant of Fig. 39, according to an embodiment of the present invention;
Fig. 45 is a perspective view of the components of the ID tool of Fig. 44, according to an embodiment of the present invention;
Fig. 46 is a top view of the first end of the plunger of the ID tool, according to one embodiment of the present invention; and
Fig. 47 are method steps for charging of the orthopaedic implant of Fig. 39 with a material agent.
The exemplifications set out herein illustrate embodiments of the invention, and such exemplifications are not to be construed as limiting the scope of the invention in any manner.

### DETAILED DESCRIPTION OF THE INVENTION

### I. Porous spinal devices - laminate designs

The present invention provides a laminate method for a spinal implant or implant component, including manufacturing methods for sheet creation, bonding/assembly methods, and ways of creating tapers. Further, the present invention provides delivery of therapeutic agents through a spinal device.

The present invention addresses these issues by providing the design and method of manufacturing of a porous spinal fusion device.

### A. Materials

Material options for the spinal device include the following: implantable polymers (such as PEEK, PMMA), implantable reinforced polymers (such as carbon-fiber reinforced PEEK), implantable metals (such as titanium, titanium alloy), and implantable ceramics (such as hydroxyapatite, alumina). One or more of these materials can be combined in a given device.

### B. Overall Design

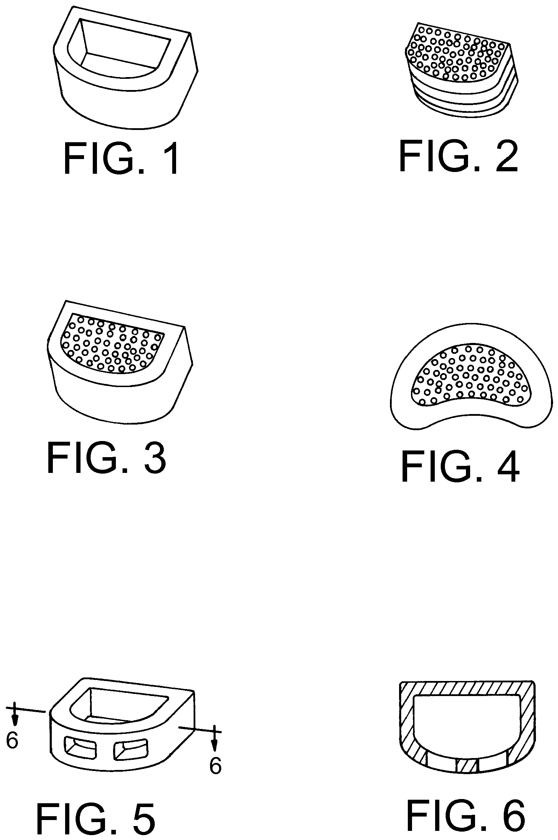

With regard to the overall design, the implant can include entirely porous material or one or more porous regions and one or more solid regions. Additionally, an entirely porous device can be created to mate with existing solid devices (See Figs. 1-3).

The porous region is created by stacking layers of material with interconnecting holes/geometry (hereafter referred to as holes).

The solid region can be formed by traditional techniques such as injection molding or machining or by bonding solid sheets together. The later method allows the solid and porous regions to be created from continuous sheets (See Fig. 4).

The holes in the sheets can be created by, for example, laser cutting, punching, etching, electrical discharge machining, plasma etching, electroforming, electron beam machining, water jet cutting, stamping, or machining. For polymer based materials, they can be created as the sheets are created by, for example, extruding, injection molding, or hot stamping.

Attachment of the sheets to each other can be achieved by any number of ways, including the following:
1. Heat. Heat can be generated by several ways:
   a. Ultrasonic welding - use ultrasonic waves to create heat at the interface of layers.
   b. Heat staking - use a heated tool to cause melting between the layers
   c. Vibratory welding
   d. Laser welding
   e. Convection - use an oven to create heat to cause bonding
   f. Intermediary layer - for example, use a material that can absorb energy waves that pass through the polymer (for example PEEK) without causing damage. The absorbed energy will cause localized heating. An example of such a coating is Clearweld by Gentex® Corporation. The laser waves that Clearweld absorbs pass through the PEEK without causing damage, allowing the layers to be melted together without large scale damage to the PEEK.
2. Chemical.
   a. Adhesives - a secondary material (such as adhesive) can be used to bond the material.
   b. Solvent bonding - a material in which the polymer or reinforced polymer is soluble can be applied to the sheet surfaces allowing multiple surfaces to be bonded to one another.
   c. Overmolding - overmolding of the polymer or reinforced polymer can provide a chemical bonding
3. Mechanical.
   a. Overmolding - overmolding of a polymer or reinforced polymer can create a mechanical lock between components on a micro or macro scale (microscale - the molded material locks with surface asperities of the existing material. Macroscale - features such as tongue-groove connections or undercuts). The overmolded material can be a separate component from the layers or one layer can be overmolded onto another layer.
   b. Features are provided within the layers or by a separate component which provides a mechanical lock - e.g. A pin, snap lock connection, dove-tail, tongue-groove, rivet, screw and / or melting tabs to create a mechanical lock. For example, one or more rivets can connect all layers of a porous implant together. These connection features can be made of any implantable material including, but not limited to, titanium, titanium alloy, PEEK, and/or other implantable polymers. These features can also be used as radiopaque markers as is described below.
   c. Some adhesives provide a mechanical bond in addition to or instead of a chemical bond.
4. Combinations of any/all of the above methods.

If the porous and solid regions are created separately (as in Figs. 1-3), it may be desirable to bond the two together. There are several methods of achieving this bond:
1. Heat. Heat can be generated by several ways:
   a. Ultrasonic welding - use ultrasonic waves to create heat at the interface of layers.
   b. Heat staking - use a heated tool to cause melting between the layers
   c. Vibratory welding
   d. Laser welding
   e. Convection - use an oven to create heat to cause bonding
   f. Intermediary layer - for example, use a material that can absorb energy waves that pass through the polymer (for example PEEK) without causing damage. The absorbed energy will cause localized heating. An example of such a coating is Clearweld by Gentex® Corporation. The laser waves that Clearweld absorbs pass through the PEEK without causing damage, allowing the layers to be melted together without large scale damage to the PEEK.
2. Chemical.
   a. Adhesives - a secondary material (such as adhesive) can be used to bond the material.
   b. Solvent bonding - a material in which the polymer or reinforced polymer is soluble can be applied to the sheet surfaces allowing multiple surfaces to be bonded to one another.
   c. Overmolding - overmolding of the polymer or reinforced polymer can provide a chemical bonding
3. Mechanical.
   a. Overmolding - overmolding of a polymer or reinforced polymer can create a mechanical lock between components on a micro or macro scale (microscale - the molded material locks with surface asperities of the existing material. Macroscale - features such as tongue-groove connections or undercuts). The overmolded material can be a separate component from the layers or one layer can be overmolded onto another layer.
   b. Features are provided within the layers or by a separate component which provides a mechanical lock - e.g. A pin, snap lock connection, dove-tail, tongue-groove, rivet, and / or melting tabs to create a mechanical lock. For example, the porous material can attach to the windows that are typical in spinal cages or to a groove or ledge is created along the interior edge of the solid ring (see Figs. 5-10). These connection features can be made of any implantable material including, but not limited to, titanium, titanium alloy, PEEK, and/or other implantable polymers. These features can also be used as radiopaque markers as is discussed later in this disclosure.
   c. Some adhesives provide a mechanical bond in addition to or instead of a chemical bond.
4. Combinations of any/all of the above methods.

Assembly of layer to layer or one component to another (for example a porous component to a solid component) can be aided by such ways as surface modifications to improve adhesive or solvent bonding or roughened surfaces.

Figs. 5-6 illustrate a spinal cage showing windows (a cross section view is shown at the right). This is an example of a type of feature onto which the porous component can be bonded.

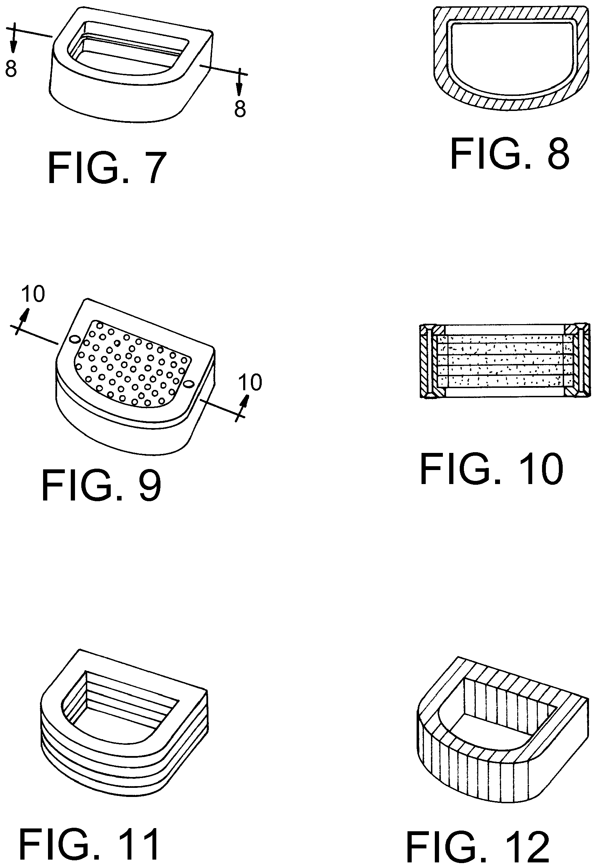

Figs. 7-8 illustrate a spinal cage showing a ledge or groove (a cross section view is shown at the right). This is an example of a type of feature onto which the porous component can be bonded.

Figs. 9-10 illustrate a spinal cage showing a two-part solid component that is assembled to contain the porous material. In this example mechanical means (screw/rivet) are used in conjunction with an adhesive bond. Adhesive ways alone, mechanical ways alone or any of the other manufacturing methods discussed in this disclosure are also options.

Figs. 11-13 illustrate a spinal cages showing laminates perpendicular, parallel, and at an angle to the axis of the implant.

The laminate portion of the implant can have layers oriented in any direction. For example, the layers can be perpendicular, parallel, or at an angle to the axis of the implant (See Figs. 11-13). This angle need not be constant within an implant.

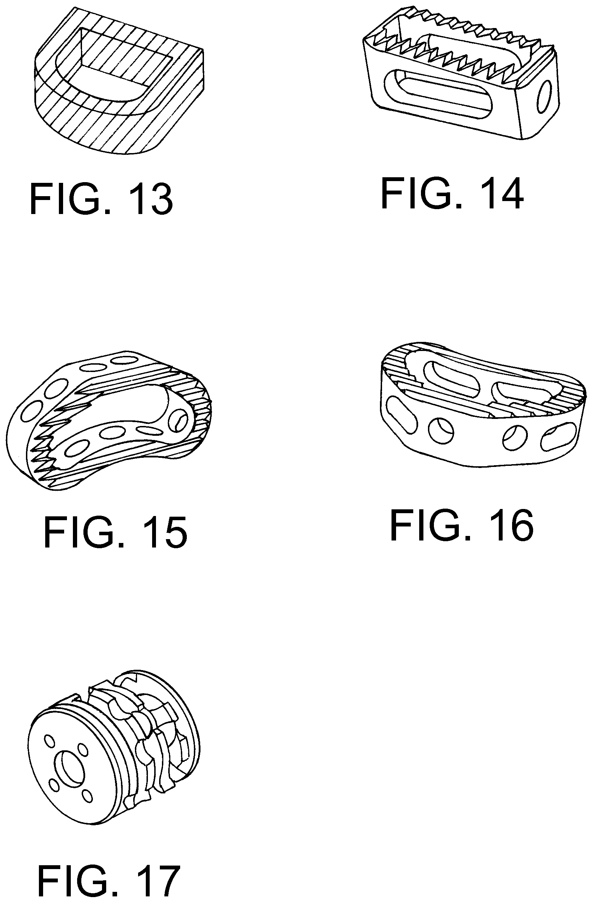

The overall shape of the implant can be of any typical existing type, such as ALIF, TLIF, PLIF, and standard round cages (see Figs. 14-17)

### C. Delivery of therapeutic agent.

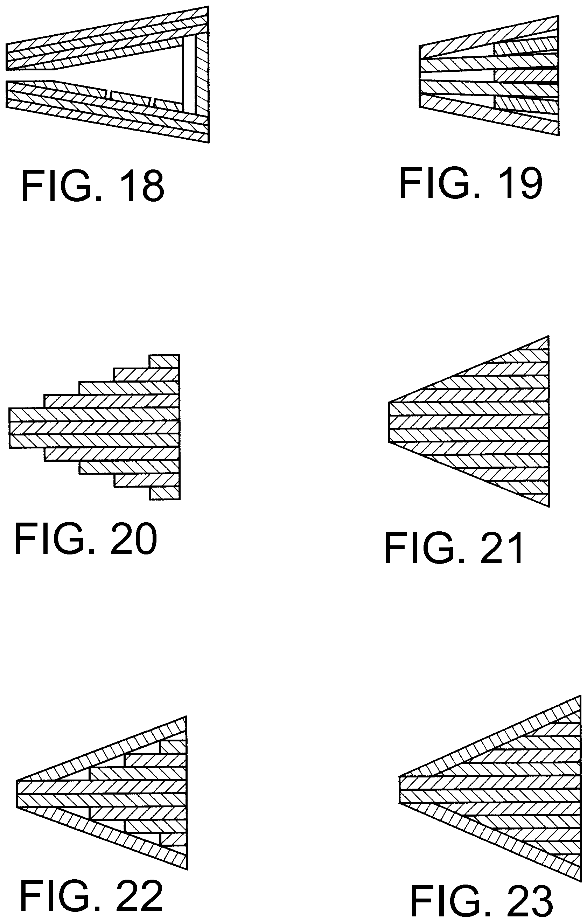

This device can be used to deliver therapeutic agents directly to the tissue surrounding the implant (See Fig. 18). Some examples of situations in which this would be desired: delivery of oncology treatments to cancerous tissue or tissue surrounding cancerous tissue; delivery of agents (such as BMP, hydroxyapatite slurry, and/or platelets) to encourage/enhance bone growth to promote faster and better fusion; and delivery of analgesic agents to reduce pain. This list is not exhaustive.

Fig. 18 illustrates a sectioned, side-view of an implant with features for the delivery of therapeutic agents.

The implant can include a reservoir for delivery of the therapeutic agent over an extended period of time. Openings leading from the reservoir to the porous material allow for controlled release of the therapeutic agents at a desired rate. The reservoir can be refilled at any time before, during, or after the surgery.

If immediate delivery of the therapeutic agents to the surrounding tissue is all that is required (not extended time release), the design need not include a reservoir. In this case, the therapeutic agents can be directly routed from the implant access to the porous material via channels. However, a reservoir can be included in an immediate delivery design; the openings in the reservoir would be sized to allow for immediate release of the therapeutic agent rather than a slower, long-term delivery.

The access in the implant (see Fig. 18) can mate with an insertion of a delivery tool (such as a needle) or a device (or catheter leading to a device) to allow for remote filling of the reservoir (such as by way of a subcutaneous port or external pain-pump).

In order to allow and promote bone growth through the implant from one vertebra to the other, openings run from the superior to the inferior portion of the implant and be appropriately sized to allow for bone ingrowth (See Fig. 18).

### D. Anterior-Posterior taper

Some implants are tapered to mate with the natural anterior-posterior taper that exists between vertebrae. If a solid portion exists, this taper can be created by traditional machining and/or molding techniques. In the porous region, there are several ways of creating this taper, including the following:
a. If the design includes a reservoir, the reservoir itself can be tapered. The porous ingrowth layers can be of uniform thickness and layered outside of the reservoir (as indicated in Fig. 18).
b. A wedge-shaped piece or pieces can create the taper with the ingrowth layers stacked on the wedge(s). This is essentially the same design as shown in Fig. 20 without the reservoir, access and holes for the therapeutic agent delivery. To allow and promote bone growth through the implant from one vertebra to the other, openings run from the superior to the inferior portion of the implant and be appropriately sized to allow for bone ingrowth (See Fig. 18).
c. Shorter layers can be stacked with larger layers to create an overall taper as in Fig. 19.
d. Layers of varying lengths can be sacked to create a stepped taper as in Fig. 20.
e. Similar to the technique in (d), layers of varying length can be stacked. A smooth taper can be created by using layers that are tapered prior to stacking or the smooth taper can be created, by such ways as machining or hot forming, after the layers are stacked. The second of these would involve first creating a part like that in (d), then removing material to create the smooth taper shown in Fig. 21.
f. Another way of creating a smooth surface on a stepped taper is to have one or more outer layers which are parallel to the taper face, as shown in Fig. 22.
g. The design in (f) does not allow for a large amount of contact area between the outer layer of the taper and the corners of the stepped layer. One way of providing increased contact area (which can provide increased strength) is to taper the stepped layers as in Fig. 21 before adding the outer layer(s) that are parallel to the face of the taper. An example of this is shown in Fig. 23.

### E. Interface with bone

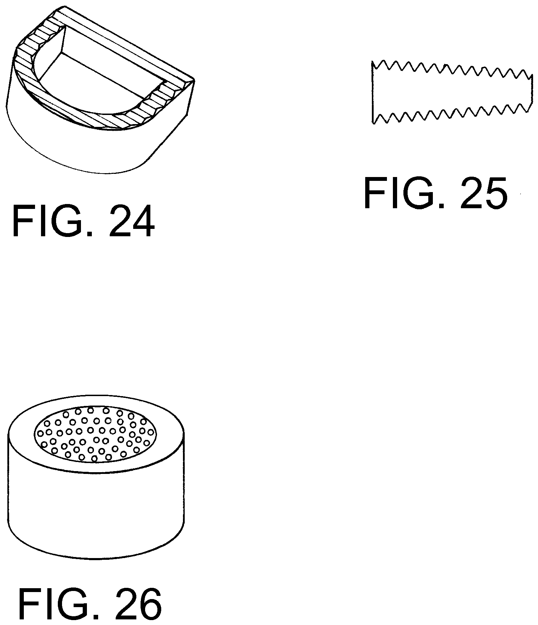

It is often desirable to have an implant-bone interface with relative high friction. Traditionally, this is achieved by such ways as a roughened implant surface, teeth (See Figs. 24-25), spikes, or hooks.

In a laminate implant, there are several options for creating such features. These options include the following:
a. Form features prior to bonding laminate sheets: Form teeth or other "rough" features into the outermost layers of the implant prior to bonding them to the other sheets. These teeth can be created by several ways:
   i. Form material - for example: heat forming, cold forming.
   ii. Remove material - for example: machining, laser cutting, chemical etching.
   iii. Add material - attach material to create the features by, for example, insert molding, mechanical attachment, adhesive bonding, laser welding, solvent bonding.
b. Form features after bonding laminate sheets: Form the rough surface features on the faces of the implant after the sheets have been bonded. These features can be formed by the same ways as listed in (a).
c. Secondary feature (such as hooks, spikes, etc) protruding from the implant into the bone. This feature can be attached by, for example, insert molding, mechanical attachment, adhesive bonding, laser welding, or solvent bonding.

Figs. 24-25 illustrate an implant showing teeth that mate with the surrounding bone.

### F. Interface with instruments

To aid in insertion of the implant into position in the body, it is often necessary to attach the implant to instrumentation. The material near the interface of the instrument and implant can often see additional stress. In a partially or fully laminate implant, it may be necessary to provide additional support in the region of this interface. This can be achieved by a number of ways, including: designing the instrument to reduce stresses and/or strengthening the implant in the region of the interface. For example, in the case of an instrument that contains a male thread which mates with a female thread in the implant, the implant can be strengthened by adding metal, solid polymer, or reinforced polymer in the region of the female thread. In machine design, thread inserts are frequently used to repair damaged threads. In this case, thread inserts can be used to strengthen the implant at the interface with the instrument(s).

### G. Radiopaque markers

When a radiolucent material, such as unfilled PEEK, is used, it is sometimes desirable to have the ability to see some or all of that implant on a diagnostic tool such as x-ray without the white-out problems of solid metal. For example, the surgeon may use such markers to determine the orientation and position of the implant to ensure proper placement during surgery. Radiopaque markers can provide this ability. The opacity and/or amount of radiopaque material can be controlled so that the marker does not prevent evaluation of the tissue near the implant by x-ray or other diagnostic ways. Material options include, but are not limited to, the following:
a. Implantable metals (stainless steel, titanium, or titanium alloys for example).
b. Barium sulfate filled PEEK.
c. Carbon filled PEEK.
d. Other polymers with radiopaque material (such as barium sulfate or zirconium dioxide).

Examples of the marker design include one or more of the following:
a. One or more radiopaque pins.
b. Assembly features such as rivets or pins.
c. Coating a portion of the device with a radiopaque material. Examples of methods for creating a radiopaque coating include, but are not limited to, the following:
   i. Using chemical vapor deposition to deposit a layer of titanium onto the polymer.
   ii. Using a radiopaque ink such as Radiopaque™ ink (developed by CI Medical).
d. One or more of the laminate layers being radiopaque. Examples of methods to make the layer(s) radiopaque include, but are not limited to, the following:
   i. Making the layer from an implantable metal (such as tantalum, titanium, titanium alloy, cobalt chrome, or stainless steel).
   ii. Using a barium sulfate filled polymer to create the layer.
   iii. Coating the layer with a radiopaque material - for example, using chemical vapor deposition to deposit a layer of titanium onto the surface of one or more layers.
e. A slightly radiopaque porous material. This can be achieved, for example, by using a polymer with barium sulfate.

### II. Porous polymer spinal fusion devices

The key to the success of a spinal fusion surgery is the formation of good bone growth between the vertebrae that are being fused. Evaluation of this bone growth is, thus, critical to determining the progress and eventual success of the surgery.

Existing porous spinal cages are made of biocompatible metals. Due to the density of these metals, the implants made post-operative examination of the tissue surrounding the implant difficult.

Several current devices are now made from solid biocompatible polymers such as PEEK. PEEK is a relatively radiolucent material. While this addresses the issue of radiopacity for solid fusion devices, it is often desired to encourage more rapid bone growth between the two vertebrae.

One solution for this problem is implants made from porous biocompatible polymers, such as PEEK or reinforced porous PEEK.

### A. Overall design

Such implants can be entirely porous or have a mix of porous and solid polymer. For example, a solid ring of material can surround a porous core (See Fig. 26).

Fig. 26 illustrates a spinal fusion device with solid region (Region 1) and porous region (Region 2)

One embodiment of the design is a porous center component that mates with existing solid, ring-like devices. This device could be assembled with the solid device in a manufacturing setting or in the operating room.

If a solid region/component exists, the porous and solid regions may need, but do not necessarily need, to be attached to one another. Examples of methods that can be used to attach the porous and solid material are:
a. Mechanical features - snap-fit connections, 'dove-tail' types of connections.
b. Adhesive bonding.
c. Solvent bonding.
d. Heat applied by, for example, laser, ultrasonic or vibratory welding, convection heating, heat staking.

### B. Material

a. Method of creating porosity
   i. Laminate design - bonding sheets of material which contain holes.
   ii. Foaming methods.
   iii. Bond 'beads' of polymer - bead of any shape can be bonded together (via, for example, heating, adhesive bonding, or solvent bonding) to create a porous structure.
   iv. Mix of polymer and dissolvable material.
      1. One method involves creating a mixture of powdered implantable material (e.g. PEEK) and a powder (e.g. salt) that is soluble in something in which the implantable material is not soluble (such as water, isopropyl alcohol for the PEEK example). The mixture is then heated to bond the implantable particles together. Pressure can also be applied to aid in the bonding of particle to particle. Heat can be created by convection or other ways (such as coating the powder with a material that absorbs a given range of energy waves - such as laser waves - and causes heating. E.g. Clearweld coating by Gentex® Corporation). Finally, dissolve away the filler to create the porous implantable material. This method can create net shape parts or raw material shapes from which individual parts can be created.
      2. Another method involves mixing an implantable polymer with a dissolvable material such as described above. The mixture is then pelletized and then injection molded to an intermediary or the final part shape. The filler is dissolved away to create the porous implantable polymer.
b. Reinforcement - If improved mechanical properties are desired, various reinforcing materials can be used. For example, carbon fiber or barium sulfate can be used.

### C. Radiopaque markers

It is sometimes desirable to have the ability to see some of the implant on a diagnostic tool such as an x-ray without the white-out problems of solid metal. For example, the surgeon may use such markers to determine the orientation and position of the implant to ensure proper placement during surgery. Radiopaque markers can provide this ability. The opacity and/or amount of radiopaque material can be controlled so that the marker does not prevent evaluation of the tissue near the implant by x-ray or other diagnostic ways. Material options include, but are not limited to, the following:
a. Implantable metals (stainless steel, titanium, or titanium alloys for example).
b. Barium sulfate filled PEEK.
c. Carbon filled PEEK.
d. Other polymers with radiopaque material (such as barium sulfate or zirconium dioxide).
Examples of the marker design include one or more of the following:
a. One or more radiopaque pins.
b. Coating a portion of the device with a radiopaque material. Examples of methods for creating a radiopaque coating include, but are not limited to, the following:
   i. Using chemical vapor deposition to deposit a layer of titanium onto the polymer.
   ii. Using a radiopaque ink such as Radiopaque™ ink (developed by CI Medical).
c. A slightly radiopaque porous material. This can be achieved, for example, by using a polymer with barium sulfate.

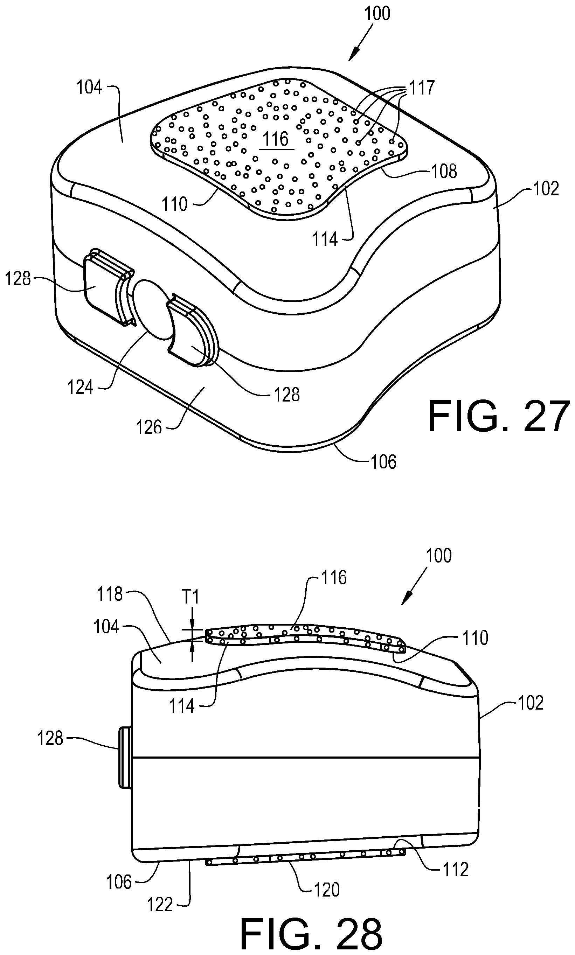

Referring now to Figs. 27-29, an embodiment of an orthopaedic implant 100 according to the present invention is shown that includes an implant body 102 formed from a substantially non-porous material having a first surface 104 and a second surface 106 opposite the first surface 104. As used herein, "substantially non-porous" indicates a porosity of 5% or less, so that the implant body 102 is mostly solid. The implant body 102 can be formed from a variety of different materials that are biocompatible and commonly used to form orthopaedic implants, including polyether ether ketone (PEEK), other polyaryl ether ketones (PAEKs), titanium, stainless steel, cobalt chrome, ultra-high molecular weight polyethylene (UHMWPE), or any previously described material. It should be appreciated that these materials are exemplary only and other biocompatible materials could be used to form the implant body. As shown in Figs. 27-29, the implant body 102 is formed in the shape of a cervical cage for spinal applications, but other shapes can also be used, as shown further herein. The first surface 104 and second surface 106 can be curved, as shown, or can be formed as planar surfaces that are substantially flat. Alternatively, one of the surfaces 104, 106 can be formed as a surface with one or more curvatures while the other surface is planar.

A cavity 108 is formed in the implant body 102 extending through the first surface 104 and second surface 106 to form a continuous cavity 108 through the implant body 102. The cavity 108 has a first cavity entrance 110 formed through the first surface 104 and a second cavity entrance 112 (shown in Fig. 28) formed through the second surface 106. One or both of the cavity entrances 110, 112 can be concentrically formed through their respective surface 104, 106 so that the cavity entrances 110, 112 have a perimeter shape that approximately matches a perimeter shape of their respective surface 104, 106, with the cavity entrances 110, 112 having a smaller perimeter than their respective surfaces 104, 106. The cavity 108 can be formed to have a constant or varying shape throughout.

A load bearing member 114 comprising a substantially porous material having a first contact surface 116 is held within the cavity 108 that is formed within the implant body 102. As used herein, "substantially porous" indicates a porosity of at least 20%, but can be significantly higher. For example, the load bearing member 114 can have a total volume, that is the entire volume occupied by the load bearing member 114, of which 60% or more is defined by pores 117 formed in the load bearing member 114. In other words, 40% of the total volume of the load bearing member 114 can be occupied by structural material forming the load bearing member 114 while 60% of the total volume is occupied by empty spaced defined by the pores 117, in aggregate. If an extremely porous material is used to form the load bearing member 114, the pores 117, in aggregate, can occupy 80% or more of the total volume of the load bearing member 114. If desired, one or more therapeutic agents can be held within some or all of the pores 117 for elution into surrounding anatomic features after implantation of the orthopaedic implant 100 to increase the efficacy of the surgical procedure. A non-exhaustive list of possible therapeutic agents that can be provided in the pores 117 includes various growth factors, bone morphogenetic factors, bone morphogenetic proteins, anti-microbial agents, anti-inflammatories, anti-coagulants, painkillers, cytotoxic substances, stem cells, and any other substance, known or unknown, that is desirable to elute from the orthopaedic implant 100 following implantation. The material(s) used to form the load bearing member 114 should, like the implant body 102, be biocompatible so that the orthopaedic implant 100 is suitable for implantation at an anatomical site within a patient. It is also useful if the load bearing member 114 is formed from one or more materials that are non-resorbable, i.e., the material of the load bearing member 114 can maintain at least 90% of its original mass after being implanted in a living patient for at least a year. Examples of such materials are PEEK, tantalum, and titanium, but other porous materials are also contemplated as being used. The load bearing member 114 can comprise either a synthetic material, such as those previously described, or one or more naturally derived materials, such as a bone graft. The naturally derived material can also be, for example, cells or tissues harvested from the patient or a different organism, scaffolds created using collagen or other biomaterials, etc. It is useful, but not required, for the load bearing member 114 to substantially fill the cavity 108 so that at least 90% of the empty space in the implant body 102 defined by the cavity 108 is filled by the bearing member 114. Such filling of the cavity 108 by the load bearing member 114 makes it easier to hold the load bearing member 114 within the cavity 108 during implantation.

The first surface 104 defines a first peak 118, which is a point on the first surface 104 that has a maximum height, relative to a ground surface, when the second surface 106 of the implant body 102 is laid on the ground surface. The first peak 118 of implant body 102 is best shown in Fig. 28, where it can be seen that the first peak 118 is adjacent to the first cavity entrance 110. With further reference to Fig. 28, it can be seen that the first contact surface 116 of the load bearing member 114 extends out of the cavity 108 past the first cavity entrance 110 so that the first contact surface 116 extends past the first peak 118, i.e., the first contact surface 116 is proud of the first surface 104. In this sense, the first contact surface 116 defines a thickness T1 that extends past and projects from the first surface 104, which can be either constant or varying throughout the first contact surface 116. By extending the first contact surface 116 past the first peak 118 of the first surface 104, the first contact surface 116 can be placed in contact with an anatomic structure, such as a vertebrae, during implantation while isolating the first surface 104 from contact with the anatomic structure. Once implanted, the porous load bearing member 114 can then bear load from the anatomic structure while allowing for ingrowth of tissue into the load bearing member 114 through the pores 117.

Due to the varying shapes of anatomic structures and desired load bearing characteristics, the first contact surface 116 can be a curved surface or a planar surface. The relative sizing between the first surface 104 and the first contact surface 116 can also be adjusted, as desired, to balance the load bearing characteristics of the load bearing member 114. As can be seen, the first contact surface 116 defines a contact surface area and the first surface 104 defines a first surface area, with the contact surface area and first surface area together defining a top surface area of the orthopaedic implant 100. The relative percentage of the top surface area that the contact surface area makes up can be altered to give varying amount of contact surface for anatomic structures during implantation. It is contemplated that the contact surface area can be 40 to 90% of the total surface area when a large contact surface 116 is desired, or less than 40% of the total surface area when a smaller contact surface 116 is desired. It should be understood that the term "top surface area" is used for convenience of description only and not to limit the scope of the present invention.

Optionally, the load bearing member 114 can have a second contact surface 120 extending out of the cavity 108 past the second cavity entrance 112 so that it extends past a second peak 122 of the second surface 106 of the implant body 102. The second peak 122 of the second surface 106 is analogous to the first peak 118 of the first surface 104, with the key difference being that the second peak 122 defines a maximum height of the second surface 106 relative to a ground surface when the first surface 104 is laid on the ground surface. The second contact surface 120 can be configured and altered similarly to the first contact surface 116 so that the second contact surface 120 can be in contact with an anatomic structure following implantation. The second contact surface 120 can be a mirror image of the first contact surface 116 or a different configuration, depending on the desired load bearing characteristics of the load bearing member 114 caused by loads bearing on the first and second contact surfaces 116, 120 from surrounding anatomic structures. It can be useful if the pores 117 of the load bearing member 114 interconnect from the first contact surface 116 to the second contact surface 120 so that a travel path through the entirety of the load bearing member 114 can be formed through interconnected pores 117 formed therein.

To assist in implanting the orthopaedic implant 100, an opening 124 can be formed through another surface 126 of the implant body 102 to the cavity 108. The opening 124 can be any size or shape that allows for an insertion tool (not shown) to be placed within the opening 124 to help steady and position the orthopaedic implant 100 during implantation. The load bearing member 114 can partially extend into the opening 124, another material can be held in the opening 124, or the opening 124 can provide a clear path to the load bearing member 114 held in the cavity 108. In a similar manner, one or more protrusions 128 can be placed adjacent to the opening 124 that are shaped to interact with the insertion tool and provide a more stable connection between the orthopaedic implant 100 and the insertion tool. The opening 124 and protrusion(s) 128 can also be configured so that a removal tool (not shown), rather than an insertion tool, can interact with the opening 124 and protrusion(s) 128 to remove the orthopaedic implant 100 from a patient following implantation, if necessary.

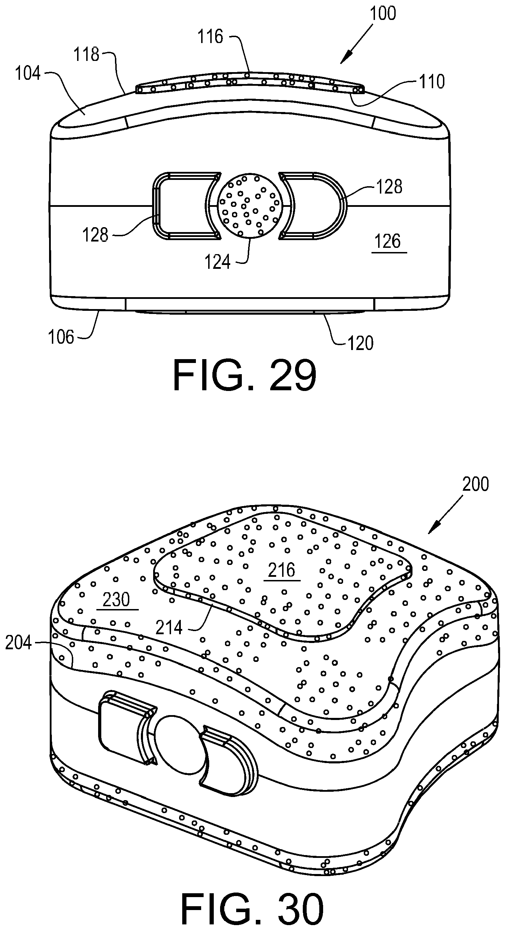

Referring now to Figs. 30-31, another embodiment of an orthopaedic implant 200 is shown that is configured similarly to orthopaedic implant 100 previously described. For brevity of description, all features of orthopaedic implant 200 that are analogous to features of orthopaedic implant 100 are numbered similarly but raised by 100. As can be seen, the first surface 204 of the implant body 202 is covered by an ingrowth material 230, shown as a porous endplate. The ingrowth material 230 can cover all or part of the first surface 204 to encourage ingrowth of surrounding tissues into the ingrowth material 230 following implantation and provide good integration of the orthopaedic implant 200. The ingrowth material 230 can be formed of any material that encourages ingrowth of a desired body tissue into the ingrowth material 230. A non-exhaustive list of contemplated materials includes porous titanium, tantalum, hydroxyapatite, tricalcium phosphate, PEEK, PAEK, polymethyl methacrylate (PMMA), polylactic acid (PLA), and polyglycolic acid (PGA), but it should be understood that many other types of materials can be used as the ingrowth material 230. Since the load bearing member 214 will initially bear the brunt of the load from surrounding anatomic structures, the ingrowth material 230 can be formed of a lower strength material, with a higher porosity than the load bearing member 214, or both. For example, the load bearing member 214 can be formed of a reinforced PEEK material that has a porosity of 60% and the ingrowth material 230 can be formed of a PEEK material that has a porosity of 80%. This allows for orthopaedic implant 200 to have a higher strength material of the load bearing member 214 initially bear the brunt of the load from surrounding anatomic structures while a higher porosity material of the ingrowth material 230 allows for better tissue ingrowth to fixate the orthopaedic implant 200.

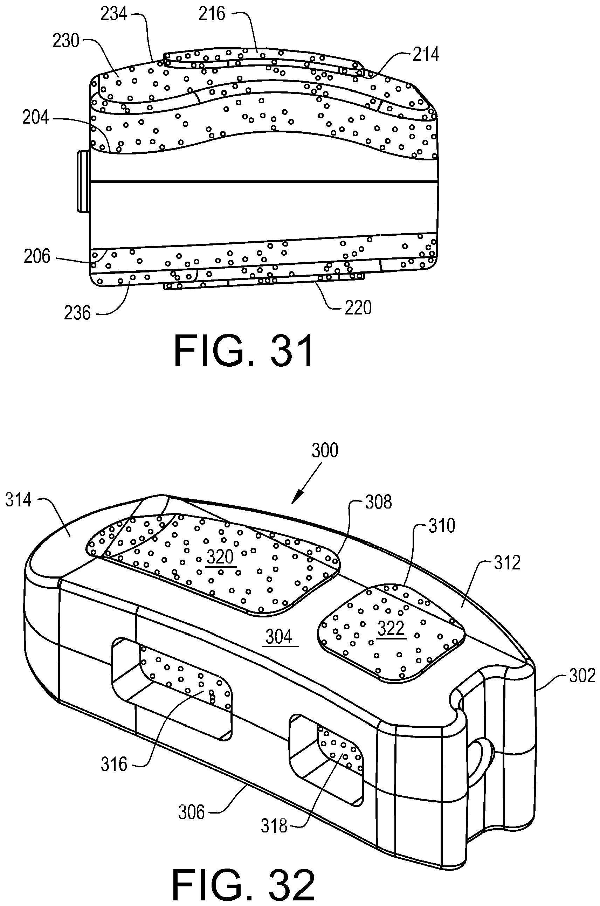

As shown in Fig. 31, the ingrowth material 230 has an ingrowth peak 234, which is the highest point of the ingrowth material 230 relative to a ground surface when the implant body 202 rests its second surface 206 on the ground surface. The first contact surface 216 of the load bearing member 214 extends out of the cavity 208 formed in the implant body 202 past the ingrowth peak 234, so that the first contact surface 216 can bear load from an anatomic structure following implantation and isolate the ingrowth material 230 from initially bearing load from the anatomic structure. The orthopaedic implant 200 can have a second ingrowth material 236 covering all or part of the second surface 206 of the implant body 202 and the load bearing member 214 can have a second contact surface 220 extending past the second ingrowth material 236 similarly to how the first ingrowth material 230 extends past the ingrowth peak 234 of the ingrowth material 230. In this sense, the ingrowth materials 230, 236 have surfaces that are analogous to the first and second surfaces 104, 106 of orthopaedic implant 100 and which the load bearing member 214 extends past.

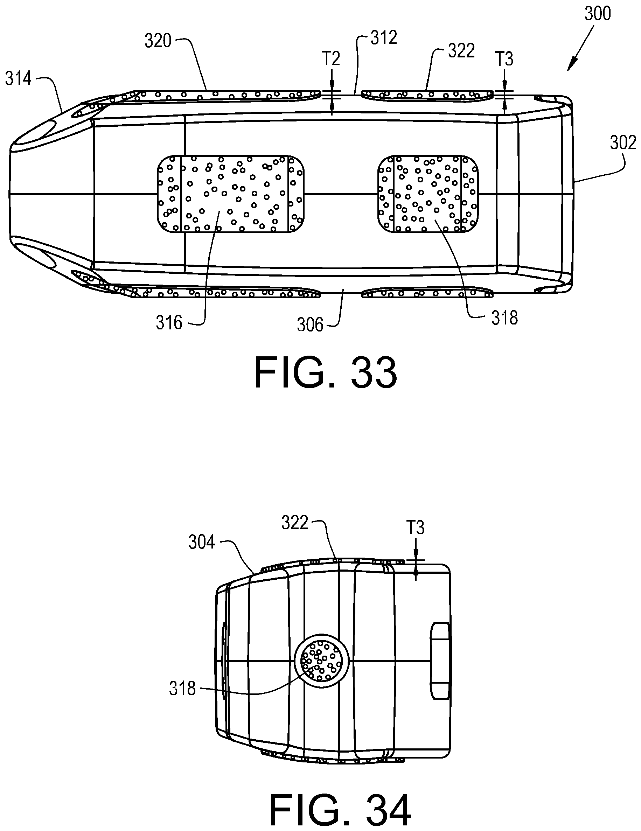

Referring now to Figs. 32-34, another embodiment of an orthopaedic implant 300 according to the present invention is shown that includes an implant body 302 configured to be used as a lumbar cage. The implant body 302 is comprised of a substantially non-porous material and has a first surface 304; a second surface 306 opposite the first surface 304; a first cavity 308 formed through the first surface 304 and second surface 306; and a second cavity 310 formed through the first surface 304 and second surface 306. As can be seen, the implant body 302 has a planar portion 312 that is flat and a curved portion 314 that has a sloped curvature. The cavities 308, 310 can be formed through the first and second surface 304, 306 all or partially within either the planar portion 312 or curved portion 314. A first load bearing member 316 is held within the first cavity 308 and a second load bearing member 318 is held within the second cavity 310. The first load bearing member 316 has a first contact surface 320 and the second load bearing member 318 has a third contact surface 322 that each extend out of their respective cavity 308, 310 past the plane of the planar portion 312, so that the contact surfaces 320, 322 can bear load from surrounding anatomic features following implantation. The load bearing members 316, 318 and their contact surfaces 320, 322 can be configured similarly to previously described load bearing members 114, 214, and even though the load bearing members 316, 318 are shown as having different sizes and total volumes, their size and total volume could be equal. The contact surfaces 320, 322 each define a respective thickness T2, T3 relative to the planar portion 312 of the first surface 304. The thicknesses T2, T3 of the contact surfaces 320, 322 can be equal to each other or could be different to provide different load bearing characteristics. For example, it may be desirable to provide load bearing member 316 with a thicker contact surface 320 than the contact surface 322 of load bearing member 318 due to the larger overall volume of load bearing member 316, in which case T2 would be greater than T3. It is also contemplated that the load bearing members 316 and 318 can be formed of different materials, have differing porosities, or be otherwise configured differently from one another to produce a desired healing effect.

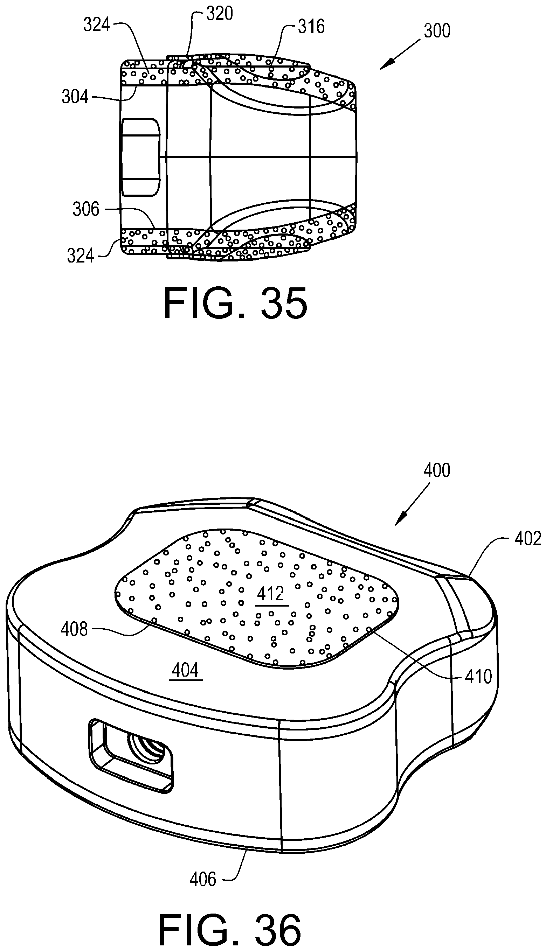

Referring now to Fig. 35, the orthopaedic implant 300 shown in Figs. 32-34 is shown with ingrowth material 324 covering the first and second surfaces 304, 306 of the implant body 302. The ingrowth material 324 can be configured in an analogous manner to previously described ingrowth material 230.

Referring now to Figs. 36-37, another embodiment of an orthopaedic implant 400 according to the present invention is shown. The orthopaedic implant 400 includes an implant body 402, configured as an anterior lumbar interbody fusion cage, comprising a substantially non-porous material having a first surface 404, a second surface 406 opposite the first surface 404, and a cavity 408 that extends through the first surface 404 and second surface 406. As can be seen, the first surface 404 is a sloped planar surface that slopes downward from a front of the implant body 402 toward a back of the implant body 402. It should be appreciated that the slope of the first surface 404 can be adjusted, as desired, to provide a variety of shapes for the implant body 402 that are suitable for different surgical procedures.

A load bearing member 410 comprising a substantially porous material is held within the cavity 408. The load bearing member 410 has a first contact surface 412 that extends out of the cavity 408 and is proud of a portion of the first surface 404 to which the first contact surface 412 is immediately adjacent. Put another way, the first contact surface 412 outwardly projects from the cavity 408 so that it will contact surrounding anatomic features when the orthopaedic implant 400 is implanted and isolate portions of the first surface 404 immediately adjacent to the cavity 408 from initially bearing load from the surrounding anatomic features. Since the first surface 404 is sloped, the first contact surface 412 does not necessarily extend past a peak of the first surface 404, as previously described first contact surfaces do. However, in all other aspects, load bearing member 410 and first contact surface 412 can be configured similarly to previously described load bearing members and contact surfaces.

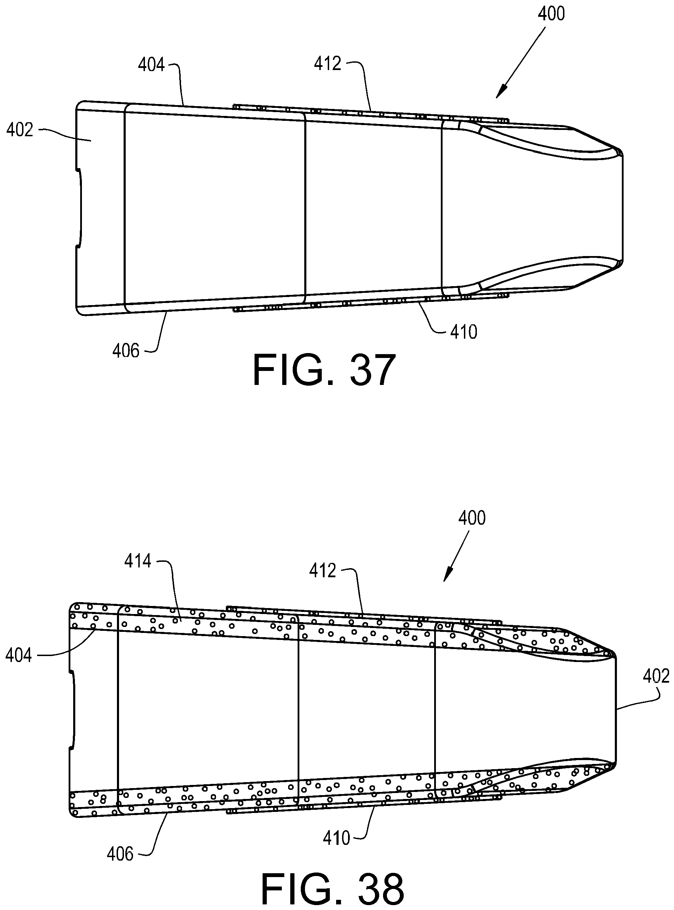

Referring now to Fig. 38, the orthopaedic implant 400 shown in Figs. 36-37 is shown with an ingrowth material 414 covering the first surface 404 of the implant body 402. The ingrowth material 414 can be configured similarly to previously described ingrowth materials. As can be seen, the load bearing member 410 is proud of a portion of the ingrowth material 414 similarly to how the load bearing member 410 shown in Figs. 36-37 is proud of a portion of the first surface 404.

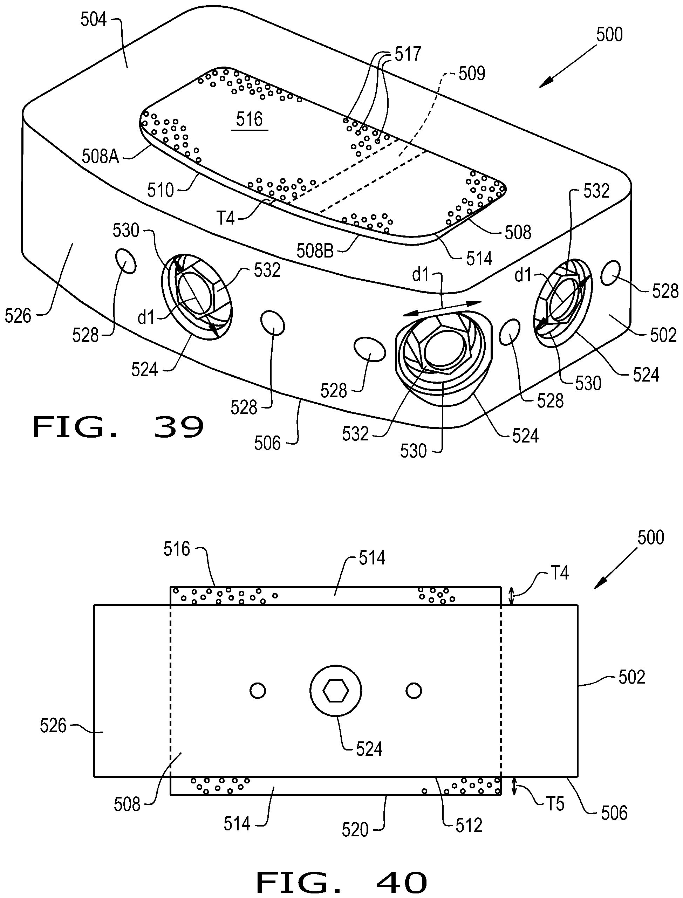

Referring now to Fig. 39, another embodiment of an orthopaedic implant 500 according to the present invention is shown. The orthopaedic implant 500 includes an implant body 502 formed from a substantially non-porous material having a first surface 504 and a second surface 506 opposite the first surface 504. The first surface 504 and second surface 506 can be curved, as shown, or can be formed as planar surfaces that are substantially flat. Alternatively, one of the surfaces 504, 506 can be formed as a surface with one or more curvatures while the other surface is planar.

A cavity 508 is formed in the implant body 502 extending through the first surface 504 and second surface 506 to form a continuous cavity 508 through the implant body 502. The cavity 508 has a first cavity entrance 510 formed through the first surface 504 and a second cavity entrance 512 (Fig. 40) formed through the second surface 506. One or both of the cavity entrances 510, 512 can be concentrically formed through their respective surface 504, 506 so that the cavity entrances 510, 512 have a perimeter shape that approximately matches a perimeter shape of their respective surface 504, 506, with the cavity entrances 510, 512 having a smaller perimeter than their respective surfaces 504, 506. The cavity 508 can be formed to have a constant or varying shape throughout.

A load bearing member 514 comprising a substantially porous material having a first contact surface 516 and having pores 517 is held within the cavity 508 that is formed within the implant body 502. The load bearing member 514 and its contact surface 516 can be configured similarly to previously described load bearing members 114, 214 and 410 and their respective contact surfaces 116, 216, and 412.

Preferably, and as illustrated, the first contact surface 516 of the load bearing member 514 extends out of the cavity 508 past the first cavity entrance 510 so that the first contact surface 516 extends past the first surface 504. In this sense, the first contact surface 516 defines a thickness T4 that extends past and projects from the first surface 504, which can be either constant or varying throughout the first contact surface 516. However, in another embodiment, the thickness T4 has a constant value of zero, thus the first contact surface 516 is flush with the first surface 504. By extending the first contact surface 516 past the first surface 504, the first contact surface 516 can be placed in contact with an anatomic structure, such as a vertebra, during implantation while isolating the first surface 504 from contact with the anatomic structure. Once implanted, the porous load bearing member 514 can then bear load from the anatomic structure while allowing for ingrowth of tissue into the load bearing member 514 through the pores 517.

One or more first openings 524 can be formed through another surface 526 of the implant body 502 to the cavity 508. The load bearing member 514 can partially extend into the first openings 524, another material can be held in the first openings 524, or the first openings 524 can provide a clear path to the load bearing member 514 held in the cavity 508. The first openings 524 can be any size or shape that allows for an insertion/delivery tool, to be described more fully below, to be placed within a first opening 524 (e.g., coupled to the first opening 524) for delivery of a material agent to the load bearing member 514 via the first opening 524 during a surgical procedure for implanting the orthopaedic implant 500. Preferably, the first openings 524 are circular openings having a threaded outer portion 530 with a diameter d₁, however the scope of the present invention covers first openings 524 having one or more different diameters or different geometric shapes with associated geometric parameters (e.g., parameters describing ellipses, squares, rectangles), and may have outer portions 530 that are not threaded.

Additionally, one or more second openings 528 can be placed adjacent to the first openings 524, shaped to interact with the insertion/delivery tool (described below) for providing a more stable connection between the orthopaedic implant 500 and the insertion/delivery tool for delivery of the material agent to the load bearing member 514 via the first opening 524 and/or for positioning the orthopaedic implant 500 during implantation. The first openings 524 and the second openings 528 can also be configured so that the insertion/delivery tool can interact with the first opening 524 and the second openings 528 to charge the load bearing member 514 with a material agent (described more fully below), or to recharge the load bearing member 514 during a second surgical procedure, if necessary.

The orthopaedic implant 500 includes plugs 532 that are coupled to the first openings 524. The plugs 532 prevent the material agent of the load bearing member 514, particularly after the load bearing member 514 has been charged with the material agent from the insertion/delivery tool via the first openings 524, from flowing out of the load bearing member 514 via the first openings 524 once the insertion /delivery tool has finished charging the load bearing member 514 and/or has been de-coupled from the first openings 524.

In another embodiment, the cavity 508 may optionally be divided into two or more sub-cavities. By way of an exemplary embodiment, the cavity 508 may optionally be divided into two sub-cavities 508A and 508B by a divider 509, formed from the same substantially non-porous material of the implant body 502. The load bearing member 514 may include porous material of different porosities held within the two sub-cavities 508A and 508B. In one embodiment, one or more first openings 524 are formed through the third surface 526 to each of the sub-cavities 508A and 508B.

Fig. 40 illustrates another view of the orthopaedic implant 500, according to an embodiment of the present invention. Optionally, the load bearing member 514 can have a second contact surface 520 extending out of the cavity 508 past the second cavity entrance 512 so that it extends past the second surface 506 of the implant body 502. The second contact surface 520 can be configured and altered similarly to the first contact surface 516 so that the second contact surface 520 can be in contact with an anatomic structure following implantation. The second contact surface 520 can be a mirror image of the first contact surface 516 or a different configuration, depending on the desired load bearing characteristics of the load bearing member 514 caused by loads bearing on the first and second contact surfaces 516, 520 from surrounding anatomic structures. It can be useful if the pores 517 of the load bearing member 514 interconnect from the first contact surface 516 to the second contact surface 520 so that a travel path through the entirety of the load bearing member 514 can be formed through interconnected pores 517 formed therein.

As illustrated, the second contact surface 520 defines a thickness T5 that extends past and projects from the second surface 506, which can be either constant or varying throughout the second contact surface 506. However, in another embodiment, the thickness T5 has a constant value of zero, thus the second contact surface 520 is flush with the second surface 506.

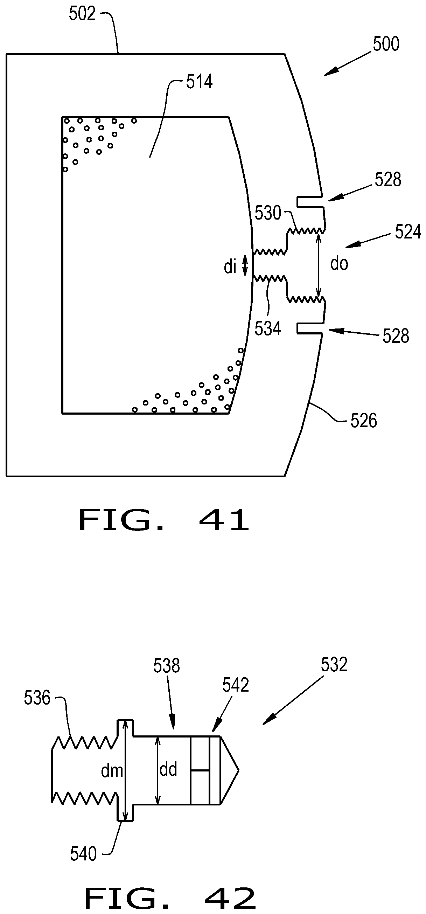

Fig. 41 illustrates a cross-sectional view of the orthopaedic implant 500, with the plug 532 removed, according to an embodiment of the present invention. For ease of illustration, only one first opening 524 is shown. The first opening 524 includes the threaded outer portion 530 and an inner portion 534, which is preferably threaded, however the scope of the invention covers the inner portion 534 being non-threaded, and having instead notches replacing the threads, or other means of removably-coupling a plug (shown in Fig. 42). The threaded outer portion 530 has an outer diameter dₒ and the threaded inner portion 534 has an inner diameter dᵢ. In one embodiment, the inner diameter dᵢ is less than the outer diameter dₒ. Reference numbers that are the same as the reference numbers of the previous figures refer to the same features.

Fig. 42 illustrates the plug 532, according to an embodiment of the present invention. The plug 532 includes a threaded proximal portion 536, a distal portion 538, and a middle portion 540 positioned between the threaded proximal portion 536 and the distal portion 538. The threaded proximal portion 536 is configured to be coupled (e.g., threaded) with the threaded inner portion 534 of the first opening 524. However, the scope of the present invention covers other means of removably-coupling a proximal portion 536 of the plug 532, in which the proximal portion 536 has, for example, ridges for removably-couling with, for example, notches of the inner portion 534. The middle portion 540 has a diameter dₘ and the distal portion 538 has a diameter d_{d}. The distal portion 538 has a distal end 542, preferably shaped for receiving a rotating means, such as a socket (not shown), for example. Although in the embodiment as illustrated, the distal end 542 is shaped as a hexagon, the scope of the present invention covers the distal end 542 formed in any shape (e.g., shaped as a cylinder containing a socket for receiving a corresponding rotating means). In one embodiment, the diameter dₘ of the middle portion 540 is less than or equal to the diameter dₒ of the threaded outer portion 530 of the first opening 524 and greater than the diameter dᵢ of the threaded inner portion 534 of the first opening 524, and the diameter d_{d} of the distal portion 538 is less than the diameter dₒ of the threaded outer portion 530 of the first opening 524 for accommodating a rotating means within the threaded outer portion 530 of the first opening 524 for threading the plug 532 into the inner portion 534 of the first opening 524 and de-threading (i.e., removing or decoupling) the plug 532 from the inner portion 534 of the first opening 524.

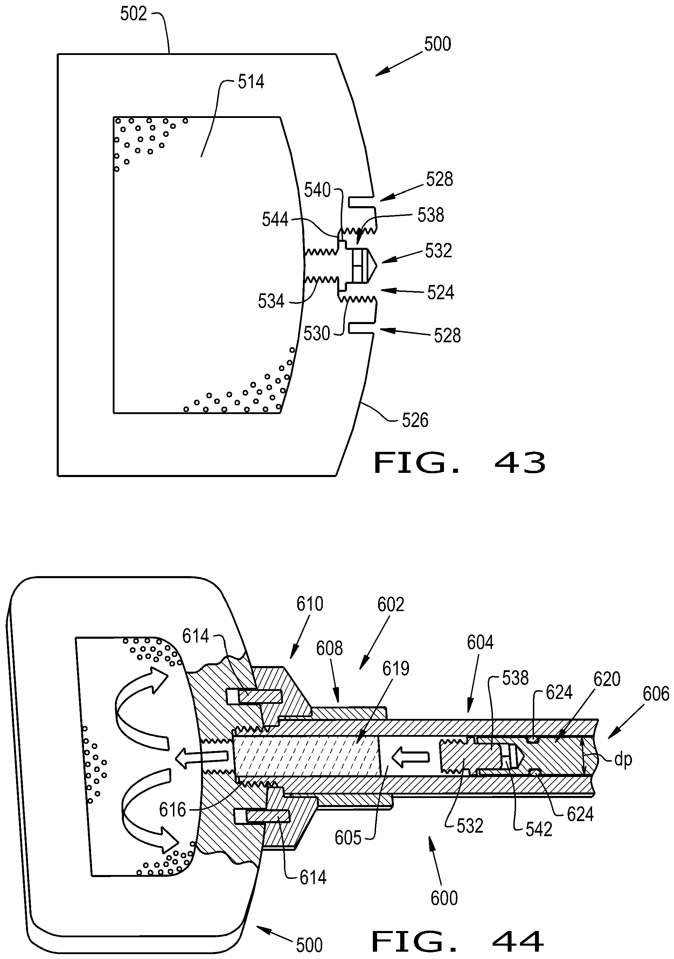

Fig. 43 illustrates a cross-sectional view of the orthopaedic implant 500, including the plug 532 coupled to the first opening 524 for sealing the first opening 524, according to an embodiment of the present invention. For ease of illustration, only one first opening 524 is shown. As illustrated, the diameters dₘ and d_{d} (shown in Fig. 42) of the middle and distal portions 540, 538, respectively, of the plug 532, are less than the diameter dₒ (shown in Fig. 41) of the threaded outer portion 530 of the first opening 524. In one embodiment, once the plug 532 is coupled in place within the first opening 524, via application of a rotating means (not shown), the middle portion 540 of the plug 532 may abut against a wall 544 separating the threaded outer portion 530 of the first opening 524 from the threaded inner portion 534 of the first opening 524. Although the coupling of the threaded proximal portion 536 with the threaded inner portion 534 of the first opening 524 may adequately seal the first opening 524 of the implant 500 from leaking a material agent contained within the load bearing member 514, the abutment of the middle portion 540 of the plug 532 against the wall 544 may assist in sealing the first opening 524, as well as preventing the plug 532 from being threaded too far into the first opening 524. In addition, as seen further below in conjunction with Fig. 44, the middle portion 540 may provide a means for a plunger 606 of an insertion/delivery tool 600 to slide the plug 532 in a cannula 604 of the insertion/delivery tool to position the plug 532 adjacent to the threaded inner portion 534, such that the plug 532 may subsequently be coupled to the threaded inner portion 534 via a rotating means, as described more fully further below.

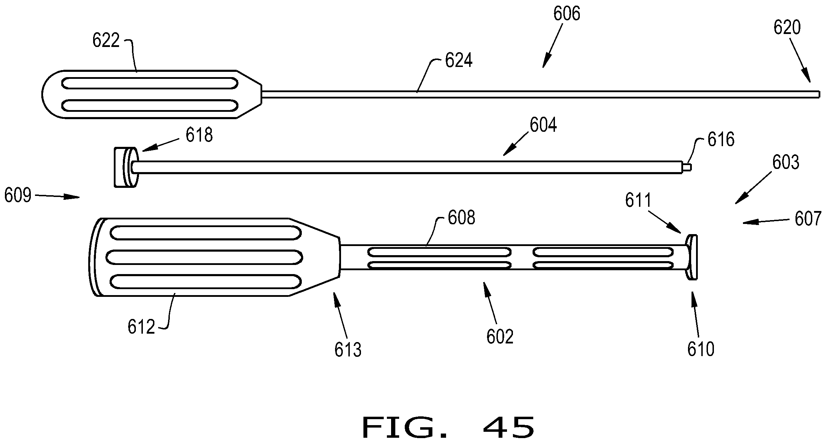

Fig. 44 illustrates a cross-sectional view of the insertion/delivery (ID) tool 600 coupled to the othopaedic implant 500 illustrated in Figs. 39-41 and Fig. 43, according to an embodiment of the present invention. The insertion delivery tool 600 is shown coupled to the orthopaedic implant 500 for ease of description. Fig. 45 illustrates several components of the ID tool 600, when the ID tool 600 is decoupled from the orthopaedic implant 500, according to an embodiment of the present invention. Reference is made to both Figs. 44 and 45 in the following description.

The ID tool 600 includes an inserter 602, a cannula 604 (i.e., a tube), and a plunger 606. In one embodiment, the inserter 602 includes a tubular portion 608, an attachment portion 610 positioned at a first end 611 of the tubular portion 608, and a handle portion 612 positioned around a second end 613 of the tubular portion 608. The attachment portion 610 may include one or more pins 614 configured to engage with (also referred to as couple with or interact with) the one or more second openings 528 for enabling the inserter 602 to insert and/or position the orthopaedic implant 500 during implant, and/or stabilize the orthopaedic implant 500 for delivery of a material agent 619 to the orthopaedic implant 500 via the first openings 524, as will be described shortly. In addition, the attachment portion 610, including the one or more pins 614 engaged with the one or more second openings 528, in combination with the tubular portion 608, provides a means for guiding the cannula 604 to the first opening 524 and stabilizing the orthopaedic implant 500 when the cannula 604 is for coupled to the first opening 524.

The cannula 604 includes a tubular passage 605 having an attachment portion 616 configured to be coupled to the orthopaedic implant 500. In one embodiment, the attachment portion 616 is threaded. The threaded attachment portion 616 is configured to thread with the outer threaded portion 530 of the first opening 524. However, the scope of the invention covers the attachment portion 616 being non-threaded, having instead other means of attachment for attaching to the outer portion 530 of the first opening 524. The cannula 604 also includes a receiving end 618 configured to receive the material agent 619 and the plug 532 coupled to a the plunger 606. The receiving end 618 may also be configured to receive a rotatable means (not shown) for rotating the cannula 604 for threading the threaded attachment end 616 with the threaded outer portion 530 of the first opening 524 for securely, but removably, coupling the cannula 604 to the orthopaedic implant body 500 (e.g., to the first opening 524). As illustrated, the inserter 602 and the cannula 604 are configured such that the cannula 604 may be positioned in the tubular portion 608 of the inserter 602 (i.e., slid inside the tubular portion 608 of the inserter 602) for coupling the cannula 604 to the orthopaedic implant 500 via the threaded outer portion 530 of the first opening 524.

The insertion/delivery tool 600 also comprises the plunger 606. The plunger 606 includes a first end 620 and a handle end 622, connected to each other via a rod 624. The first end 620 is configured to couple with the distal portion 538 of the plug 532 for pushing the plug 532, and the material agent 619 residing in the cannula 604 between the plug 532 and the attachment end 616, through the cannula 604 towards the first opening 524. In one embodiment, the first end 620 is a hexagon-shaped socket configured to couple with a hexagon-shaped distal end 542 of the plug 532. Once the first end 620 of the plunger 606 is coupled with the distal end 542 of the plug 532, a force may be applied to the handle end 622 for pushing the plug 532 (and any material agent 619 residing in the cannula 604 between the plug 532 and the attachment end 616) through the cannula 604, thereby forcing the material agent 619 into the load bearing member 514 via the first opening 524, and once the threaded proximal portion 536 of the plug 532 is pushed up against the threaded inner portion 534 of the first opening 524, the handle end 622 may be rotated for threading the plug 532 into the threaded inner portion 534 of the first opening 524, thereby sealing the first opening 524 against expulsion or leakage of the material agent 619 from the load bearing member 514. In one embodiment, the plunger 606 comprises one or more sealing rings 624, such as O-rings, positioned around the rod 624 for preventing leakage of the material agent 619 into a portion of the cannula 604 between the sealing ring 624 and the receiving end 618 of the cannula 604 as the plunger 606 is being pushed down the cannula 604 in the direction of the first opening 524.

In one embodiment, and as illustrated, the tubular passage 605 has a tubular passage diameter dₚ, and the middle diameter dₘ of the plug 532 is equal to the tubular passage diameter dₚ and the distal diameter d_{d} of the plug 532 is less than the tubular passage diameter dₚ.

In another embodiment, the inserter 602 and the cannula 604 positioned within the tubular portion 608 of the inserter 602 may collectively be referred to as a tubular assembly 603 having the tubular passage 605. The tubular assembly also includes a first end 607 configured to couple (i.e., attach) to the orthopaedic implant 500 and a second end 609 configured to receive the material agent 619, the plug 532 and the plunger 606.

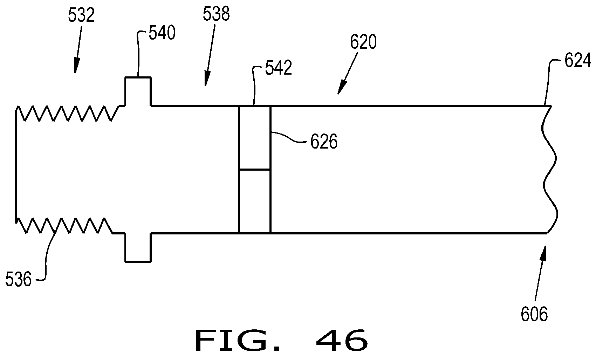

Fig. 46 illustrates the first end 620 of the plunger 606, according to another embodiment of the present invention. In this embodiment, the first end 620 is connected to the distal end 542 of the plug 532 at a joint 626, or in other words, the first end 620 includes the plug 532. In operation, once the inserter 602 and the cannula 604 have been coupled to the orthopaedic implant 500, and the material agent is placed in the receiving end 618 of the cannula 604, and the plunger 606, including the first end 620 connected to the plug 532, has pushed the threaded proximal portion 536 up against the threaded inner portion 534 of the first opening 524, the plunger 606 is rotated, causing the plug to be threaded into the threaded inner portion 534. In one embodiment, the first end 620 is connected to the distal end 542 of the plug 532 in such a manner that once the threading of the plug is complete, thereby sealing the first opening 524, any further rotation of the plunger 606 causes the first end 620 to break with the distal end 542 of the plug 532 along the joint 626, allowing removal of the plunger 606 from the cannula 604. In one embodiment, the distal end 542 is configured as a hexagon, or any other shape, to allow the plug 532 to be de-threaded (i.e., removed) from the first opening 524 at a later time. In another embodiment, the distal end 542 is not configured to accommodate any rotational means for removing the plug 532, and thus the plug 532 is permanently coupled to the first opening 524.

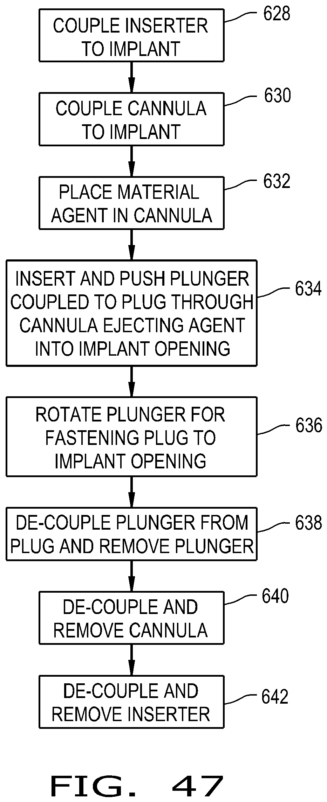

Fig. 47 shows method steps for charging of the orthopaedic implant 500 with the material agent 619.

In step 628, the inserter 602 is coupled to the orthopaedic implant 500. In one embodiment, the one or more pins 614 of the attachment portion 610 of the inserter 602 is coupled to the one or more second openings 528 of the orthopaedic implant 500. In one embodiment, the two second openings 528 are formed in the surface 526, also referred to as a side surface of the orthopaedic implant. The surface 526 is formed between the first surface 504 and the second surface 506 of the orthopaedic implant 500. In an embodiment, the two second openings are formed to straddle the first opening 524 in the surface 526.

In step 630, the cannula 604 is coupled to the orthopaedic implant 500. In one embodiment, the attachment portion 616 of the cannula 604 is slid into the inserter 602 until the attachment portion 616 reaches the outer portion 530 of the first opening 524. The cannula 604 is then rotated, preferably via use of a rotating means, such as a socket, wrench, etc., applied to the receiving end 618 of the cannula 604, causing the attachment portion 616 to rotate, thereby coupling the attachment portion 616 to the outer portion 530 of the first opening 524. In one embodiment, the both the attachment portion 616 and the outer portion 530 are threaded, and the rotation of the cannula 604 causes the threaded attachment portion 616 to thread with the threaded outer portion 530, thereby coupling the cannula 604 to the orthopaedic implant 500, or according to one embodiment, to the first opening 524 of the orthopaedic implant 500.

In step 632, the material agent 619 is placed into the cannula 604 via the receiving end 618. In one embodiment, the material agent is a flowable material. The scope of the present invention covers flowable materials, such as bone pastes, bone putties, bone grafts, or therapeutics, for example, antibiotics, blood plasmas, bone marrow, pain medications, or drugs, such as tumor-fighting drugs, all having conventional viscosities known to those of skill in the art. Furthermore, the scope of the present invention covers any biological material agents that may be embodied as a flowable material. In addition, the scope of the present invention covers material agents having a range of viscosities, which in combination with a porous load bearing member 514 having various interconnectivities and pore sizes, are deliverable into the porous load bearing member 514 via the first opening 524, the cannula 604 and the plunger 606.

In step 634, the first end 620 of the plunger 606 coupled to the plug 532 is inserted into and pushed through the cannula 604 in the direction of the first opening 524, thereby enabling the plug 532 to push the material agent 619 in the direction of the first opening 524 and forcing the material agent 619 through the first opening 524 for charging the porous load bearing member 514 of the orthopaedic implant 500 with the material agent 619. In one embodiment, the first end 620 of the plunger 606 is removeably-coupled to the distal end 542 of the plug 532, via for example, a socket. In another embodiment, the first end 620 of the plunger 606 is breakably-coupled to the distal end 542 of the plug 532 at a joint 626 between the distal portion 542 and the first end 620.

In step 636, after the plunger 606 coupled to the plug 532 has pushed the proximal portion 536 of the plug 532 adjacent to the inner portion 534 of the first opening 524, the plunger 606 is rotated within the cannula 604, causing the plug 532 to couple (also referred to as attach or fasten) with the first opening 524, thereby sealing the first opening 524. In one embodiment, both the proximal portion 536 of the plug 532 and the inner portion 534 of the first opening 524 are threaded, and the rotation of the cannula 604 causes the proximal portion 536 to thread with the inner portion 534, thereby sealing the first opening 524 against expulsion (also referred to as leakage) of the material agent 619 of the load bearing member 514 through the first opening 524.

In step 638, if the first end 620 of the plunger 606 is removably-coupled to the distal end 542 of the plug 532, then after the plug 532 is threaded into the first opening 524 in step 636, the first end 620 of the plunger 606 is decoupled from the distal end 542 and the plunger 606 may be removed from the cannula 604. If the first end 620 of the plunger 606 is breakably-coupled to the distal end 542 of the plug 532 at the joint 626, then after the plug 532 is threaded into the first opening 524 in step 636, further rotation of the plunger 606 causes the first end 620 to break with the distal end 542 of the plug 532 along the joint 626. The plunger 606 may then be removed from the cannula 604.

In step 640, the cannula 604 is rotated within the inserter 602 in the opposite direction as in step 630, thereby de-coupling the cannula 602 from the orthopaedic implant 500. For example, rotating the cannula 604 in the opposite direction unthreads the threaded attachment end 616 of the cannula 604 from the threaded outer portion 530 of the first opening 524, thereby allowing the cannula 604 to be slid out of the tubular portion 608 of the inserter 602.

In step 642, the inserter 602 is de-coupled from the orthopaedic implant 500. In one embodiment, the one or more pins 614 of the attachment portion 610 of the inserter 602 is decoupled from the one or more second openings 528 of the orthopaedic implant 500.

While this invention has been described with respect to at least one embodiment, the present invention can be further modified within the scope of this disclosure. This application is therefore intended to cover any variations, uses, or adaptations of the invention using its general principles. Further, this application is intended to cover such departures from the present disclosure as come within known or customary practice in the art to which this invention pertains and which fall within the limits of the appended claims.

## Claims

1. An orthopaedic implant (500), comprising:
an implant body (502) having a first surface (504), a second surface (506) opposite said first surface (504), and a cavity (508) formed therein that extends through said first surface (504) and said second surface (506), said implant body (502) being substantially non-porous, said implant body (502) further including a third surface (526) with at least one first opening (524) formed therethrough to said cavity (508), said at least one first opening (524) comprising an outer portion (530) having an outer diameter (dₒ) and an inner portion (534) having an inner diameter (dᵢ), said outer diameter (dₒ) greater than said inner diameter (dᵢ); and
a load bearing member (514) comprising a substantially porous material held within said cavity (508), said substantially porous material configured to receive, via said at least one first opening (524), a material agent (619), said load bearing member (514) having a first contact surface (516) extending out of said cavity (508) past said first surface (504),
wherein said outer portion (530) of said at least one first opening (524) is configured to couple to a tool (600) for receiving, from the tool (600), said material agent (619), and
wherein said inner portion (534) of said at least one first opening (524) is configured to couple to a plug (532) for preventing said material agent (619), once received by said substantially porous material via said at least one first opening (524) from said tool (600), from exiting said substantially porous material via said at least one first opening (524).

2. The orthopaedic implant (500) according to claim 1, wherein said load bearing member (514) has a second contact surface (520) opposite said first contact surface (516), said load bearing member (514) having interconnecting pores (517) extending from said first contact surface (516) to said second contact surface (520), said load bearing member (514) having a total volume and said interconnecting pores (517) in aggregate occupying at least 60% of said total volume.

3. The orthopaedic implant (500) according to claim 1 or 2, wherein said cavity comprises at least two sub-cavities (508A, 508B), wherein said at least one first opening comprises at least two first openings (524), and wherein said substantially porous material held within said at least two sub-cavities (508A, 508B) has different porosities.

4. The orthopaedic implant (500) according to any of claims 1 to 3, wherein said material agent (619) is a flowable material, wherein preferably said flowable material is a bone graft or a bone putty or a therapeutic.

5. The orthopaedic implant (500) according to any of claims 1 to 4, wherein said inner portion (534) of said at least one first opening (524) comprises a threaded inner portion (534), plug (532) comprises a threaded proximal portion (536) threaded with said threaded inner portion (534) of said at least one first opening (524) and a distal portion (538) having a diameter (d_{d}) less than said first diameter (dₒ) of said outer portion (530) of said at least one first opening (524), said outer portion (530) comprising a threaded outer portion (530).

6. The orthopaedic implant (500) according to claim 5, wherein said plug (532) comprises a middle portion (540), said middle portion (540) having a diameter (dₘ) greater than said second diameter (di) of said threaded inner portion (534) of said at least one first opening (524) and less than said first diameter (dₒ) of said threaded outer portion (530) of said at least one first opening (524); or
wherein said distal portion (538) has a distal end (542), said distal end (542) shaped for receiving a rotating means for unthreading said plug (532) from said at least one first opening (524) and said distal end (542) being preferably shaped as a hexagon.

7. The orthopaedic implant (500) according to any of claims 1 to 6, wherein said inner portion (534) of said at least one first opening (524) is configured to couple to said plug (532) received from said tool (600); or
wherein said third surface (526) comprises a least one second opening (528) configured to couple with said tool (600).

8. The orthopaedic implant (500) according to any of claims 1 to 7, further comprising said material agent (619) and said plug (532), wherein said substantially porous material of said load bearing member (514) includes said material agent (619), and wherein said inner portion (534) of said at least one first opening (524) is coupled to said plug (532), said plug (532) sealing said at least one first opening (524).

9. A tool (600) configured for use with an orthopaedic implant (500), said orthopaedic implant (500) including an implant body (502) having a first surface (504), a second surface (506) opposite said first surface (504), a cavity (508) formed therein that extends through said first surface (504) and said second surface (506), said implant body (502) being substantially non-porous, said implant body (502) further including a third surface (526) with at least one first opening (524) formed therethrough to said cavity (508) and at least one second opening (528), said at least one first opening (524) comprising an threaded outer portion (530) having an outer diameter (dₒ) and an threaded inner portion (534) having an inner diameter (dᵢ), said outer diameter (dₒ) greater than said inner diameter (dᵢ), and a load bearing member (514) comprising a substantially porous material held within said cavity, said load bearing member (514) configured to receive, via said at least one first opening, a material agent (619), said load bearing member (514) having a first contact surface (516) extending out of said cavity (508) past said first surface (504), said tool (600) comprising:
a tubular assembly (603) including a tubular passage (605) having a first end (607) and a second end (609), wherein said first end (607) comprises a means for attachment to said implant body (502);
a plug (532); and
a plunger (606) coupled to said plug (532),
wherein said tubular passage (605) is configured to receive, via said second end (609), said material agent (619) and said plunger (606) coupled to said plug (532),
wherein said plunger (606) coupled to said plug (532) is configured to slide through said tubular passage (605) for expelling said material agent (619) from said tubular passage (605) into said load bearing member (514) via said at least one first opening (524), and wherein
said plunger (606) coupled to said plug (532) is configured to rotate within said tubular passage (605) for coupling said plug (532) with said first opening (524) to seal said first opening (524) against expulsion of said material agent (619) from said load bearing member (514) via said first opening (524).

10. The tool (600) according to claim 9, wherein said tubular assembly (603) comprises an inserter (602) including a tubular portion (608) and a cannula (604) positioned within the tubular portion (608) of the inserter (602), wherein said means of attachment of said tubular assembly (603) includes an attachment portion (610) of said inserter (602) and a threaded attachment portion (616) of said cannula (604), said attachment portion (610) of said inserter (602) having at least one pin (614) configured to couple with said at least one second opening (528) of said third surface (526) of said implant body (502), said threaded attachment portion (616) of said cannula (604) configured to couple with said threaded outer portion (530) of said at least one first opening (524) of said third surface (526), and wherein said cannula (604) includes said tubular passage (605),
wherein, in particular, said inserter (602) includes a handle portion (612) positioned around said tubular portion (608).

11. The tool (600) according to claim 9, wherein said plug (532) comprises a proximal threaded portion (536) configured to couple to said inner threaded portion (534) of said at least one first opening (524), a distal portion (538), said plunger (606) coupled to said distal portion (538) and said distal portion (538) having a distal diameter (d_{d}) less than said outer diameter (dₒ) of said threaded outer portion (530) of said at least one first opening (524), and a middle portion (540) having a middle diameter (dₘ) greater than said inner diameter (di) of said threaded inner portion (534) of said at least one first opening (524) and less than or equal to said outer diameter (dₒ) of said threaded outer portion (530).

12. The tool (600) according to claim 11, wherein said tubular passage (605) has a tubular passage diameter (dₚ), and wherein said middle diameter (dₘ) of said plug (532) is equal to said tubular passage diameter (dₚ), and wherein said distal diameter (d_{d}) of said plug (532) is less than said tubular passage diameter (dₚ); or
wherein said distal portion (538) of said plug (532) comprises a distal end portion, and wherein said plunger (606) is breakably-coupled to said distal end portion at a joint such that when said plunger (606) is rotated within said tubular passage (605) for coupling said proximal threaded portion (536) to said inner threaded portion (534) of said at least one first opening (524) for sealing said at least one first opening (524), said plunger (606) breaks from said distal end portion at said joint; or
wherein said distal portion (538) of said plug (532) comprises a distal end portion formed as a head, and wherein said plunger (606) has a first end (620) configured as a socket, and wherein said first end (620) of said plunger (606) is coupled to said distal end portion of said plug (532).

13. The tool (600) according to any of claims 9 to 12, wherein said plunger (606) has a first end (620) coupled to said plug (532), a handle end (622), and a rod (624) connecting the handle end (622) to the first end (620), wherein said rod (624) includes at least one sealing ring (624).

14. The tool (600) according to any of claims 9 to 13, wherein said material agent (619) is a flowable material, wherein preferably said flowable material is a bone graft or a bone putty or a therapeutic.

15. A method of charging an orthopaedic implant (500) with a material agent (619) before surgery, said orthopaedic implant (500) including an implant body (502) having a first surface (504), a second surface (506) opposite said first surface (504), a cavity (508) formed therein that extends through said first surface (504) and said second surface (506), said implant body (502) being substantially non-porous, said implant body (502) further including a third surface (526) with at least one first opening (524) formed therethrough to said cavity (508) and at least one second opening (528), said at least one first opening (524) comprising an threaded outer portion (530) having an outer diameter (dₒ) and an threaded inner portion (534) having an inner diameter (dᵢ), said outer diameter (dₒ) greater than said inner diameter (dᵢ), and a load bearing member (514) comprising a substantially porous material held within said cavity (508), said load bearing member (514) configured to receive, via said at least one first opening (524), said material agent (619), said load bearing member (514) having a first contact surface (516) extending out of said cavity (508) past said first surface (504), said method comprising:
coupling a tubular assembly (603) to said third surface (526) of said implant body (502), wherein said tubular assembly (603) includes a tubular passage (605) having a first end (607) and a second end (609), and wherein said first end (607) is coupled to said third surface (526) such that said tubular passage (605) is centered on said at least one first opening (524);
placing said material agent (619) into said tubular passage (605) via said second end (609);
sliding a plunger (606) coupled to a plug (532) through said tubular passage (605) via said second end (609), thereby expelling said material agent (619) from said tubular passage (605) into said load bearing member (514) via said at least one first opening (524); and
rotating said plunger (606) within said tubular passage (605) for coupling said plug (532) with said at least one first opening (524) for sealing said at least one first opening (524) against expulsion of said material agent (619) from said load bearing member (514) via said first opening (524).

## Patentansprüche

1. Orthopädisches Implantat (500), umfassend:
einen Implantatkörper (502) mit einer ersten Oberfläche (504), einer zweiten Oberfläche (506) gegenüber der ersten Oberfläche (504) und einer darin gebildeten Kavität (508), welche sich durch die erste Oberfläche (504) und die zweite Oberfläche (506) erstreckt, wobei der Implantatkörper (502) im Wesentlichen nicht porös ist, wobei der Implantatkörper (502) ferner eine dritte Oberfläche (526) mit mindestens einer sich durch diese hindurch zu der Kavität (508) erstreckenden ersten Öffnung (524) umfasst, wobei die mindestens eine erste Öffnung (524) einen äußeren Abschnitt (530) mit einem Außendurchmesser (dₒ) und einen inneren Abschnitt (534) mit einem Innendurchmesser (dᵢ) umfasst, wobei der Außendurchmesser (dₒ) größer als der Innendurchmesser (dᵢ) ist; und
eine tragenden Komponente (514), umfassend ein im Wesentlichen poröses Material, das in der Kavität (508) aufgenommen ist, wobei das im Wesentlichen poröse Material dazu ausgebildet ist, ein Materialmittel (619) über die mindestens eine erste Öffnung (524) aufzunehmen, wobei die tragende Komponente (514) eine erste Kontaktoberfläche (516) aufweist, die sich aus der Kavität (508) über die erste Oberfläche (504) hinaus erstreckt,
wobei der äußere Abschnitt (530) der mindestens einen ersten Öffnung (524) dazu ausgebildet ist, mit einem Werkzeug (600) verbindbar zu sein, zum Aufnehmen des Materialmittels (619) von dem Werkzeug (600), und
wobei der innere Abschnitt (534) der mindestens einen ersten Öffnung (524) dazu ausgebildet ist, mit einem Stopfen (532) verbindbar zu sein, um zu verhindern, dass das Materialmittel (619), wenn es von dem im Wesentlichen porösen Material über die mindestens eine erste Öffnung (524) von dem Werkzeug (600) aufgenommen wurde, von dem im Wesentlichen porösen Material über die mindestens eine erste Öffnung (524) austritt.

2. Orthopädisches Implantat (500) gemäß Anspruch 1, wobei die tragende Komponente (514) eine zweite Kontaktoberfläche (520) gegenüber der ersten Kontaktoberfläche (516) aufweist, wobei sich die tragende Komponente (514) mit verbindenden Poren (517) von der ersten Kontaktoberfläche (516) zu der zweiten Kontaktoberfläche (520) erstreckt, wobei die tragende Komponente (514) ein Gesamtvolumen aufweist und die verbindenden Poren (517) im Ganzen mindestens 60 % von dem Gesamtvolumen in Anspruch nehmen.

3. Orthopädisches Implantat (500) gemäß Anspruch 1 oder 2, wobei die Kavität mindestens zwei Unterkavitäten (508A, 508B) umfasst, wobei die mindestens eine erste Öffnung mindestens zwei erste Öffnungen (524) umfasst, und wobei das im Wesentlichen poröse Material, das in den mindestens zwei Unterkavitäten (508A, 508B) gehalten wird, unterschiedliche Porositäten aufweist.

4. Orthopädisches Implantat (500) gemäß einem der Ansprüche 1 bis 3, wobei das Materialmittel (619) ein fließfähiges Material ist, wobei das fließfähige Material bevorzugt ein Knochengewebe oder ein Knochenkitt oder ein Therapeutikum ist.

5. Orthopädisches Implantat (500) gemäß einem der Ansprüche 1 bis 4, wobei der innere Abschnitt (534) der mindestens einen ersten Öffnung (524) einen mit einem Gewinde versehenen inneren Abschnitt (534) umfasst, wobei der Stopfen (532) einen mit einem Gewinde versehenen proximalen Abschnitt (536) umfasst, der mit dem mit einem Gewinde versehenen inneren Abschnitt (534) der mindestens einen ersten Öffnung (524) verschraubt ist, und einen distalen Abschnitt (538) umfasst, der einen Durchmesser (d_{d}) aufweist, der kleiner als der erste Durchmesser (d₀) des äußeren Abschnitts (530) der mindestens einen ersten Öffnung (524) ist, umfasst, wobei der äußere Abschnitt (530) einen mit einem Gewinde versehenen äußeren Abschnitt (530) umfasst.

6. Orthopädisches Implantat (500) gemäß Anspruch 5, wobei der Stopfen (532) einen mittleren Abschnitt (540) umfasst, wobei der mittlere Abschnitt (540) einen Durchmesser (dₘ) aufweist, der größer als der zweite Durchmesser (dᵢ) des mit einem Gewinde versehenen inneren Abschnitts (534) der mindestens einen ersten Öffnung (524) und kleiner als der erste Durchmesser (dₒ) des mit einem Gewinde versehenen äußeren Abschnitts (530) der mindestens einen ersten Öffnung (524) ist; oder
wobei der distale Abschnitt (538) ein distales Ende (542) aufweist, wobei das distale Ende (542) geformt ist zum Aufnehmen eines Drehhilfsmittels zum Abschrauben des Stopfen (532) von der mindestens einen ersten Öffnung (524) und das distale Ende (542) bevorzugt als ein Sechskant geformt ist.

7. Orthopädisches Implantat (500) gemäß einem der Ansprüche 1 bis 6, wobei der innere Abschnitt (534) der mindestens einen ersten Öffnung (524) konfiguriert ist zum Koppeln an den Stopfen (532), aufgenommen von dem Werkzeug (600); oder
wobei die dritte Oberfläche (526) mindestens eine zweite Öffnung (528), konfiguriert zum Koppeln mit dem Werkzeug (600), umfasst.

8. Orthopädisches Implantat (500) gemäß einem der Ansprüche 1 bis 7, ferner umfassend das Materialmittel (619) und den Stopfen (532), wobei das im Wesentlichen poröse Material der tragenden Komponente (514) das Materialmittel (619) einschließt, und wobei der innere Abschnitt (534) der mindestens einen ersten Öffnung (524) an den Stopfen (532) gekoppelt ist, wobei der Stopfen (532) die mindestens eine erste Öffnung (524) verschließt.

9. Werkzeug (600), das zur Verwendung mit einem orthopädischen Implantat (500) ausgebildet ist, wobei das orthopädische Implantat (500) einen Implantatkörper (502) umfasst, mit einer ersten Oberfläche (504), einer zweiten Oberfläche (506) gegenüber der ersten Oberfläche (504), einer darin gebildeten Kavität (508), welche sich durch die erste Oberfläche (504) und die zweite Oberfläche (506) hindurch erstreckt, wobei der Implantatkörper (502) im Wesentlichen nicht porös ist, wobei der Implantatkörper (502) ferner eine dritte Oberfläche (526) umfasst, mit mindestens einer sich durch diese hindurch zu der Kavität (508) erstreckenden ersten Öffnung (524), und mindestens einer zweiten Öffnung (528), wobei die mindestens eine erste Öffnung (524) einen mit einem Gewinde versehenen äußeren Abschnitt (530) mit einem Außendurchmesser (dₒ) und einen mit einem Gewinde versehenen inneren Abschnitt (534) mit einem Innendurchmesser (dᵢ) umfasst, wobei der Außendurchmesser (dₒ) größer als der Innendurchmesser (dᵢ) ist, und eine tragende Komponente (514), umfassend ein im Wesentlichen poröses Material, das in der Kavität (508) aufgenommen ist, wobei die tragende Komponente (514) dazu ausgebildet ist, ein Materialmittel (619) über die mindestens eine erste Öffnung (524) aufzunehmen, wobei die tragende Komponente (514) eine erste Kontaktoberfläche (516) aufweist, die sich aus der Kavität (508) über die erste Oberfläche (504) hinaus erstreckt, wobei das Werkzeug (600) umfasst:
eine rohrförmige Anordnung (603), umfassend einen rohrförmigen Durchgang (605) mit einem ersten Ende (607) und einem zweiten Ende (609), wobei das erste Ende (607) ein Hilfsmittel zum Anbringen an den Implantatkörper (502) umfasst;
einen Stopfen (532); und
einen Kolben (606), gekoppelt an den Stopfen (532),
wobei der rohrförmige Durchgang (605) dazu ausgebildet ist, das Materialmittel (619) und den mit dem Stopfen (532) gekoppelten Kolben (606) über das zweite Ende (609) aufzunehmen,
wobei der mit dem Stopfen (532) gekoppelte Kolben (606) dazu ausgebildet ist, durch den rohrförmigen Durchgang (605) zu gleiten, um das Materialmittel (619) von dem rohrförmigen Durchgang (605) über die mindestens eine erste Öffnung (524) in die tragende Komponente (514) auszustoßen, und wobei
der mit dem Stopfen (532) gekoppelte Kolben (606) dazu ausgebildet ist, in dem rohrförmigen Durchgang (605) zu rotieren, um den Stopfen (532) mit der ersten Öffnung (524) zu koppeln, um die erste Öffnung (524) gegen Ausstoßen des Materialmittels (619) von der tragenden Komponente (514) über die erste Öffnung (524) abzudichten.

10. Werkzeug (600) gemäß Anspruch 9, wobei die rohrförmige Anordnung (603) einen Inserter (602), einschließend einen rohrförmigen Abschnitt (608) und eine Kanüle (604), positioniert in dem rohrförmigen Abschnitt (608) des Inserters (602), einschließt, wobei das Hilfsmittel zum Anbringen der rohrförmigen Anordnung (603) einen Anbringungsabschnitt (610) des Inserters (602) und einen mit einem Gewinde versehenen Anbringungsabschnitt (616) der Kanüle (604) einschließt, wobei der Anbringungsabschnitt (610) des Inserters (602) mindestens einen Stift (614), konfiguriert zum Koppeln mit der mindestens einen zweiten Öffnung (528) der dritten Oberfläche (526) des Implantatkörpers (502), aufweist, wobei der mit einem Gewinde versehene Anbringungsabschnitt (616) der Kanüle (604) konfiguriert ist zum Koppeln mit dem mit einem Gewinde versehenen äußeren Abschnitt (530) der mindestens einen ersten Öffnung (524) der dritten Oberfläche (526), und wobei die Kanüle (604) den rohrförmigen Durchgang (605) einschließt,
wobei insbesondere der Inserter (602) einen Griffabschnitt (612), positioniert um den rohrförmigen Abschnitt (608), einschließt.

11. Werkzeug (600) gemäß Anspruch 9, wobei der Stopfen (532) umfasst einen proximalen mit einem Gewinde versehenen Abschnitt (536), konfiguriert zum Koppeln an den inneren mit einem Gewinde versehenen Abschnitt (534) der mindestens einen ersten Öffnung (524), einen distalen Abschnitt (538), den Kolben (606), gekoppelt an den distalen Abschnitt (538), und wobei der distale Abschnitt (538) einen distalen Durchmesser (d_{d}) aufweist, der kleiner ist als der Außendurchmesser (dₒ) des mit einem Gewinde versehenen äußeren Abschnitts (530) der mindestens einen ersten Öffnung (524), und einen mittleren Abschnitt (540) mit einem mittleren Durchmesser (dₘ), der größer ist als der Innendurchmesser (dᵢ) des mit einem Gewinde versehenen inneren Abschnitts (534) der mindestens einen ersten Öffnung (524) und kleiner ist als der oder gleich ist dem Außendurchmesser (dₒ) des mit einem Gewinde versehenen äußeren Abschnitts (530).

12. Werkzeug (600) gemäß Anspruch 11, wobei der rohrförmige Durchgang (605) einen rohrförmiger Durchgang-Durchmesser (dₚ) aufweist, und wobei der mittlere Durchmesser (dₘ) des Stopfens (532) gleich dem rohrförmiger Durchgang-Durchmesser (dₚ) ist, und wobei der distal Durchmesser (d_{d}) des Stopfens (532) kleiner als der rohrförmiger Durchgang-Durchmesser (dₚ) ist; oder
wobei der distale Abschnitt (538) des Stopfens (532) einen distalen Endabschnitt umfasst, und wobei der Kolben (606) trennbar gekoppelt ist an den distalen Endabschnitt bei einer Verbindungsstelle, so dass, wenn der Kolben (606) in dem rohrförmigen Durchgang (605) zum Koppeln des proximalen mit einem Gewinde versehenen Abschnitts (536) an den inneren mit einem Gewinde versehenen Abschnitt (534) der mindestens einen ersten Öffnung (524) zum Verschließen der mindestens einen ersten Öffnung (524) gedreht wird, sich der Kolben (606) von dem distalen Endabschnitt an der Verbindungsstelle trennt; oder
wobei der distale Abschnitt (538) des Stopfens (532) einen distalen Endabschnitt, gebildet als ein Kopf, umfasst, und wobei der Kolben (606) ein erstes Ende (620), konfiguriert als eine Hülse, aufweist, und wobei das erste Ende (620) des Kolbens (606) gekoppelt ist an den distalen Endabschnitt des Stopfens (532).

13. Werkzeug (600) gemäß einem der Ansprüche 9 bis 12, wobei der Kolben (606) ein erstes Ende (620), gekoppelt an den Stopfen (532), ein Griffende (622) und eine Stange (624), die das Griffende (622) mit dem ersten Ende (620) verbindet, aufweist, wobei die Stange (624) mindestens einen ersten Dichtungsring (624) einschließt.

14. Werkzeug (600) gemäß einem der Ansprüche 9 bis 13, wobei das Materialmittel (619) ein fließfähiges Material ist, wobei das fließfähige Material bevorzugt ein Knochengewebe oder ein Knochenkitt oder ein Therapeutikum ist.

15. Verfahren zum Beschicken eines orthopädischen Implantats (500) mit einem Materialmittel (619) vor einem chirurgischen Eingriff, wobei das orthopädische Implantat (500) einen Implantatkörper (502) umfasst, mit einer ersten Oberfläche (504), einer zweiten Oberfläche (506) gegenüber der ersten Oberfläche (504), einer darin gebildeten Kavität (508), welche sich durch die erste Oberfläche (504) und die zweite Oberfläche (506) hindurch erstreckt, wobei der Implantatkörper (502) im Wesentlichen nicht porös ist, wobei der Implantatkörper (502) ferner eine dritte Oberfläche (526) umfasst, mit mindestens einer sich durch diese hindurch zu der Kavität (508) erstreckenden ersten Öffnung (524), und mindestens einer zweiten Öffnung (528), wobei die mindestens eine erste Öffnung (524) einen mit einem Gewinde versehenen äußeren Abschnitt (530) mit einem Außendurchmesser (dₒ) und einen mit einem Gewinde versehenen inneren Abschnitt (534) mit einem Innendurchmesser (dᵢ) umfasst, wobei der Außendurchmesser (dₒ) größer als der Innendurchmesser (dᵢ) ist, und eine tragende Komponente (514), umfassend ein im Wesentlichen poröses Material, das in der Kavität (508) aufgenommen ist, wobei die tragende Komponente (514) dazu ausgebildet ist, ein Materialmittel (619) über die mindestens eine erste Öffnung (524) aufzunehmen, wobei die tragende Komponente (514) eine erste Kontaktoberfläche (516) aufweist, die sich aus der Kavität (508) über die erste Oberfläche (504) hinaus erstreckt,, wobei das Verfahren umfasst:
Koppeln einer rohrförmigen Anordnung (603) an die dritte Oberfläche (526) des Implantatkörpers (502), wobei die rohrförmige Anordnung (603) einen rohrförmigen Durchgang (605) mit einem ersten Ende (607) und einem zweiten Ende (609) umfasst, und wobei das erste Ende (607) derart an die dritte Oberfläche (526) gekoppelt ist, dass der rohrförmige Durchgang (605) an der mindestens einen ersten Öffnung (524) zentriert wird;
Anordnen des Materialmittels (619) in dem rohrförmigen Durchgang (605) über das zweite Ende (609);
Gleiten eines mit einem Stopfen (532) verbundenen Kolbens (606) durch den rohrförmigen Durchgang (605) über das zweite Ende (609), wodurch das Materialmittel (619) von dem rohrförmigen Durchgang (605) über die mindestens eine erste Öffnung (524) in die tragenden Komponente (514) ausgestoßen wird; und
Rotieren des Kolbens (606) in dem rohrförmigen Durchgang (605) zum Koppeln des Stopfens (532) mit der mindestens einen ersten Öffnung (524) zum Abdichten der mindestens einen ersten Öffnung (524) gegen ein Ausstoßen des Materialmittels (619) von der tragenden Komponente (514) über die erste Öffnung (524).

## Revendications

1. Un implant orthopédique (500), comprenant :
un corps d'implant (502) ayant une première surface (504), une deuxième surface (506) opposée à ladite première surface (504), et une cavité (508) formée dans celui-ci qui s'étend à travers ladite première surface (504) et ladite deuxième surface (506), ledit corps d'implant (502) étant sensiblement non-poreux, ledit corps d'implant (502) comprenant en outre une troisième surface (526) avec au moins une première ouverture (524) formée à travers celle-ci jusqu'à ladite cavité (508), ladite au moins une première ouverture (524) comprenant une partie extérieure (530) ayant un diamètre extérieur (dₒ) et une partie intérieure (534) ayant un diamètre intérieur (dᵢ), ledit diamètre extérieur (dₒ) étant supérieur audit diamètre intérieur (dᵢ) ; et
un élément porteur de charge (514) comprenant un matériau sensiblement poreux maintenu à l'intérieur de ladite cavité (508), ledit matériau sensiblement poreux étant configuré pour recevoir, via ladite au moins une première ouverture (524), un agent de matériau (619), ledit élément porteur de charge (514) ayant une première surface de contact (516) s'étendant hors de ladite cavité (508) au-delà de ladite première surface (504) ;
dans lequel ladite partie extérieure (530) de ladite au moins une première ouverture (524) est configurée pour se coupler à un outil (600) pour recevoir, de l'outil (600), ledit agent de matériau (619), et
dans lequel ladite partie intérieure (534) de ladite au moins une première ouverture (524) est configurée pour se coupler à un bouchon (532) pour empêcher ledit agent de matériau (619), une fois reçu par ledit matériau sensiblement poreux via ladite au moins une première ouverture (524) dudit outil (600), de sortir dudit matériau sensiblement poreux via ladite au moins une première ouverture (524).

2. Implant orthopédique (500) selon la revendication 1, dans lequel ledit élément porteur de charge (514) a une deuxième surface de contact (520) opposée à ladite première surface de contact (516), ledit élément porteur de charge (514) ayant des pores d'interconnexion (517) s'étendant de ladite première surface de contact (516) à ladite deuxième surface de contact (520), ledit élément porteur de charge (514) ayant un volume total et lesdits pores d'interconnexion (517) en agrégats occupant au moins 60 % dudit volume total.

3. L'implant orthopédique (500) selon la revendication 1 ou 2, dans lequel ladite cavité comprend au moins deux sous-cavités (508A, 508B), dans lesquelles ladite au moins une première ouverture comprend au moins deux premières ouvertures (524), et dans lequel ledit matériau sensiblement poreux maintenu à l'intérieur desdites au moins deux sous-cavités (508A, 508B) a des porosités différentes.

4. L'implant orthopédique (500) selon l'une quelconque des revendications 1 à 3, dans lequel ledit agent de matériau (619) est un matériau fluide, dans lequel de préférence ledit matériau fluide est une greffe osseuse ou un mastic osseux ou un produit thérapeutique.

5. Implant orthopédique (500) selon l'une quelconque des revendications 1 à 4, dans lequel ladite partie intérieure (534) de ladite au moins une première ouverture (524) comprend une partie intérieure filetée (534), le bouchon (532) comprend une partie proximale filetée (536) filetée avec ladite partie intérieure filetée (534) de ladite au moins une première ouverture (524) et une partie distale (538) ayant un diamètre (d_{d}) inférieur audit premier diamètre (dₒ) de ladite partie extérieure (530) de ladite au moins une première ouverture (524), ladite partie extérieure (530) comprenant une partie extérieure filetée (530).

6. L'implant orthopédique (500) selon la revendication 5, dans lequel ledit bouchon (532) comprend une partie centrale (540), ladite partie centrale (540) ayant un diamètre (dₘ) supérieur audit second diamètre (dᵢ) de ladite partie intérieure filetée (534) de ladite au moins une première ouverture (524) et inférieur audit premier diamètre (dₒ) de ladite partie extérieure filetée (530) de ladite au moins une première ouverture (524) ; ou
dans lequel ladite partie distale (538) a une extrémité distale (542), ladite extrémité distale (542) étant formée pour recevoir un moyen de rotation pour dévisser ledit bouchon (532) de ladite au moins une première ouverture (524) et ladite extrémité distale (542) étant de préférence formée comme un hexagone.

7. Implant orthopédique (500) selon l'une quelconque des revendications 1 à 6, dans lequel ladite partie intérieure (534) de ladite au moins une première ouverture (524) est configurée pour s'accoupler audit bouchon (532) reçu dudit outil (600) ; ou
dans lequel ladite troisième surface (526) comprend au moins une deuxième ouverture (528) configurée pour s'accoupler avec ledit outil (600).

8. Implant orthopédique (500) selon l'une quelconque des revendications 1 à 7, comprenant en outre ledit agent de matériau (619) et ledit bouchon (532), dans lequel ledit matériau sensiblement poreux dudit élément porteur de charge (514) comprend ledit agent de matériau (619), et dans lequel ladite partie intérieure (534) de ladite au moins une première ouverture (524) est couplée audit bouchon (532), ledit bouchon (532) fermant ladite au moins une première ouverture (524).

9. Outil (600) configuré pour être utilisé avec un implant orthopédique (500), ledit implant orthopédique (500) comprenant un corps d'implant (502) ayant une première surface (504), une seconde surface (506) opposée à ladite première surface (504), une cavité (508) formée dans celui-ci qui s'étend à travers ladite première surface (504) et ladite seconde surface (506), ledit corps d'implant (502) étant sensiblement non poreux, ledit corps d'implant (502) comprenant en outre une troisième surface (526) avec au moins une première ouverture (524) formée à travers celle-ci vers ladite cavité (508) et au moins une deuxième ouverture (528), ladite au moins une première ouverture (524) comprenant une partie extérieure filetée (530) ayant un diamètre extérieur (dₒ) et une partie intérieure filetée (534) ayant un diamètre intérieur (dᵢ), ledit diamètre extérieur (do) étant supérieur audit diamètre intérieur (dᵢ), et un élément porteur de charge (514) comprenant un matériau sensiblement poreux maintenu dans ladite cavité, ledit élément porteur de charge (514) étant configuré pour recevoir, via ladite au moins une première ouverture, un agent de matériau (619), ledit l'élément porteur de charge (514) ayant une première surface de contact (516) s'étendant hors de ladite cavité (508) au-delà de ladite première surface (504), ledit outil (600) comprenant :
un ensemble tubulaire (603) comprenant un passage tubulaire (605) ayant une première extrémité (607) et une deuxième extrémité (609), dans lequel ladite première extrémité (607) comprend un moyen de fixation audit corps d'implant (502) ;
un bouchon (532) ; et
un piston (606) couplé à audit bouchon (532),
dans lequel ledit passage tubulaire (605) est configuré pour recevoir, via ladite seconde extrémité (609), ledit agent de matériau (619) et ledit piston (606) couplé audit bouchon (532),
dans lequel ledit piston (606) couplé audit bouchon (532) est configuré pour coulisser à travers ledit passage tubulaire (605) pour expulser ledit agent de matériau (619) dudit passage tubulaire (605) dans ledit élément porteur de charge (514) via ladite au moins une première ouverture (524), et dans lequel
ledit piston (606) couplé audit bouchon (532) est configuré pour tourner à l'intérieur dudit passage tubulaire (605) pour coupler ledit bouchon (532) avec ladite première ouverture (524) afin de rendre étanche ladite première ouverture (524) contre l'expulsion dudit agent de matériau (619) hors dudit élément porteur de charge (514) par ladite première ouverture (524).

10. Outil (600) selon la revendication 9, dans lequel ledit ensemble tubulaire (603) comprend un dispositif d'insertion (602) comprenant une partie tubulaire (608) et une canule (604) positionnée à l'intérieur de la partie tubulaire (608) du dispositif d'insertion (602), dans lequel ledit moyen de fixation dudit ensemble tubulaire (603) comprend une partie de fixation (610) dudit dispositif d'insertion (602) et une partie de fixation filetée (616) de ladite canule (604), ladite partie de fixation (610) dudit dispositif d'insertion (602) ayant au moins une broche (614) configurée pour s'accoupler avec ladite au moins une deuxième ouverture (528) de ladite troisième surface (526) dudit corps d'implant (502), ladite partie de fixation filetée (616) de ladite canule (604) configurée pour s'accoupler avec ladite partie extérieure filetée (530) de ladite au moins une première ouverture (524) de ladite troisième surface (526), et dans lequel ladite canule (604) comprend ledit passage tubulaire (605),
dans lequel, en particulier, ledit dispositif d'insertion (602) comprend une partie formant poignée (612) positionnée autour de ladite partie tubulaire (608).

11. Outil (600) selon la revendication 9, dans lequel ledit bouchon (532) comprend une partie proximale filetée (536) configurée pour se coupler à ladite partie intérieure filetée (534) de ladite au moins une première ouverture (524), une partie distale (538), ledit piston (606) couplé à ladite partie distale (538) et ladite partie distale (538) ayant un diamètre distal (d_{d}) inférieur audit diamètre extérieur (dₒ) de ladite partie extérieure filetée (530) de ladite au moins une première ouverture (524), et une partie centrale (540) ayant un diamètre moyen (dₘ) supérieur audit diamètre intérieur (dᵢ) de ladite partie intérieure filetée (534) de ladite au moins une première ouverture (524) et inférieur ou égal audit diamètre extérieur (dₒ) de ladite partie extérieure filetée (530).

12. Outil (600) selon la revendication 11, dans lequel ledit passage tubulaire (605) a un diamètre de passage tubulaire (dₚ), et dans lequel ledit diamètre moyen (dm) dudit bouchon (532) est égal audit diamètre de passage tubulaire (dₚ), et dans lequel ledit diamètre distal (d_{d}) dudit bouchon (532) est inférieur audit diamètre de passage tubulaire (dₚ) ; ou:
dans lequel ladite partie distale (538) dudit bouchon (532) comprend une partie d'extrémité distale, et dans lequel ledit piston (606) est couplé de manière cassable à ladite partie d'extrémité distale au niveau d'un joint de telle sorte que lorsque ledit piston (606) est tourné à l'intérieur dudit passage tubulaire (605) pour coupler ladite partie proximale filetée (536) à ladite partie intérieure filetée (534) de ladite au moins une première ouverture (524) pour sceller ladite au moins une première ouverture (524), ledit piston (606) se casse de ladite partie d'extrémité distale au niveau dudit joint ; ou
dans lequel ladite partie distale (538) dudit piston (532) comprend une partie d'extrémité distale formée comme une tête, et dans lequel ledit piston (606) a une première extrémité (620) configurée comme une douille, et dans lequel ladite première extrémité (620) dudit piston (606) est couplée à ladite partie d'extrémité distale (538) dudit piston (532).

13. Outil (600) selon l'une quelconque des revendications 9 à 12, dans lequel ledit piston (606) a une première extrémité (620) couplée audit bouchon (532), une extrémité de poignée (622), et une tige (624) reliant l'extrémité de poignée (622) à la première extrémité (620), dans lequel ladite tige (624) comprend au moins une bague d'étanchéité (624).

14. Outil (600) selon l'une quelconque des revendications 9 à 13, dans lequel ledit agent de matériau (619) est un matériau fluide, dans lequel de préférence ledit matériau fluide est une greffe osseuse ou un mastic osseux ou un produit thérapeutique.

15. Procédé de chargement d'un implant orthopédique (500) avec un agent de matériau (619) avant une opération chirurgicale, ledit implant orthopédique (500) comprenant un corps d'implant (502) ayant une première surface (504), une seconde surface (506) opposée à ladite première surface (504), une cavité (508) formée dans celui-ci qui s'étend à travers ladite première surface (504) et ladite seconde surface (506), ledit corps d'implant (502) étant sensiblement non poreux, ledit corps d'implant (502) comprenant en outre une troisième surface (526) avec au moins une première ouverture (524) formée à travers celle-ci vers ladite cavité (508) et au moins une deuxième ouverture (528), ladite au moins une première ouverture (524) comprenant une partie extérieure filetée (530) ayant un diamètre extérieur (dₒ) et une partie intérieure filetée (534) ayant un diamètre intérieur (dᵢ), ledit diamètre extérieur (dₒ) étant plus grand que ledit diamètre intérieur (dᵢ), et un élément porteur de charge (514) comprenant un matériau sensiblement poreux maintenu à l'intérieur de ladite cavité (508), ledit élément porteur de charge (514) étant configuré pour recevoir, via ladite au moins une première ouverture (524), ledit agent de matériau (619), ledit élément porteur de charge (514) ayant une première surface de contact (516) s'étendant hors de ladite cavité (508) au-delà de ladite première surface (504), ledit procédé comprenant :
le couplage d'un ensemble tubulaire (603) à ladite troisième surface (526) dudit corps d'implant (502), dans lequel ledit ensemble tubulaire (603) comprend un passage tubulaire (605) ayant une première extrémité et une deuxième extrémité (609), et dans lequel ladite première extrémité (607) est couplée à ladite troisième surface (526) de telle sorte que ledit passage tubulaire (605) est centré sur ladite au moins une première ouverture (524) ;
le placement dudit agent de matériau (619) dans ledit passage tubulaire (605) via ladite deuxième extrémité (609) ;
le coulissement d'un piston (606) couplé à un bouchon (532) à travers ledit passage tubulaire (605) via ladite seconde extrémité (609), expulsant ainsi ledit agent de matériau (619) dudit passage tubulaire (605) dans ledit élément porteur (514) via ladite au moins une première ouverture (524) ; et
la rotation dudit piston (606) à l'intérieur dudit passage tubulaire (605) pour coupler ledit bouchon (532) avec ladite au moins une première ouverture (524) pour rendre étanche ladite au moins une première ouverture (524) contre l'expulsion dudit agent de matériau (619) hors dudit élément porteur de charge (514) par ladite première ouverture (524).
